# EUROPEAN PATENT APPLICATION

(11) **EP 4 663 762 A2**
(43) Date of publication of application: **17.12.2025**
(21) Application number: 24763300.1
(22) Date of filing: 30.04.2024
(51) Int. Cl.: C12N 15/86, C12N 15/62, A61P 31/12, A61P 35/00, C12N 5/10, C07K 19/00, A61K 35/17, A61K 35/62

(54) **IMMUNE REJECTION-RESISTANT ENGINEERED CELL**

(30) Priority: 02.03.2023 WO PCT/CN2023/079408
(71) Applicant: HongKong Bioheng Biotech Limited, Kowloon Hong Kong 999077 (HK)
(72) Inventor: ZHOU, Yali, Hong Kong 999077 (CN); CHEN, Gong, Hong Kong 999077 (CN); ZENG, Jiuqin, Hong Kong 999077 (CN); REN, Jiangtao, Hong Kong 999077 (CN)
(74) Representative: Rimini, Rebecca
(86) International application number: PCT/CN2024/090859
(87) International publication number: WO 2024/179620

(57) **Abstract**

An immunosuppressive molecule comprising an immunosuppressive protein binding domain, a transmembrane domain and a co-stimulatory domain and the molecule does not comprise a primary signaling domain, wherein the immunosuppressive protein is selected from two or more of NKG2A, TIM3, LAG3, TIGIT, CTLA4, PD1 and FasL. An engineered cell expressing the immunosuppressive molecule and a composition comprising the engineered cell. A method for reducing immune rejection using the immunosuppressive molecule.

## Description

### CROSS-REFERENCE TO RELATED APPLICATION

The present application claims the priority to the PCT international application with the international application No. PCT/CN2023/079408, and entitled "IMMUNE REJECTION-RESISTANT ENGINEERED CELL", filed on March 02, 2023, which is incorporated in the present disclosure by reference in its entirety.

### TECHNICAL FIELD

The present invention pertains to the field of immunotherapy. More specifically, the present invention relates to an immunosuppressive molecule. The present invention further relates to an engineered cell expressing the immunosuppressive molecule, and a method for suppressing immune rejection by cells of a subject.

### BACKGROUND ART

Immune rejection refers to the process by which an organism destroys a graft (allogeneic cells, tissues, or organs) through a specific immune response. Generally, it refers to that, after transplantation, the host can recognize the graft antigen and generate response, while the immune cell in the graft can also recognize the host antigen tissue and generate immune response. In allogeneic transplantation, there are two basic types of immune rejection reactions: host-versus-graft reaction (HVGR) and graft-versus-host reaction (GVHR). Clinically, HVGR is more prevalent.

Adoptive cell therapy (ACT) plays an increasingly important role in the treatment of diseases such as tumors. ACT refers to a therapy that transfers therapeutic cells (such as immune cells) into a subject to improve the function and characteristics of the immune system of the subject. The most commonly used therapeutic cell type is T cells, but other cell types, e.g. NK cells, B cells, dendritic cells, etc., have also been applied. The therapeutic cells can originate from the subject himself (i.e., autologous therapy) or from another individual of the same species (i.e., allogeneic therapy). Allogeneic therapy offers obvious advantages over autologous therapy, such as lower production costs, shorter waiting time for the subject, and a wider range of applications. However, due to reasons such as immune rejection reaction between the subject and exogenous therapeutic cells, allogeneic therapy has not yet been widely used.

In clinical application of ACT, a patient often undergoes lymphodepletion or bone marrow cell preconditioning (e.g., using high-dose systemic chemotherapy or combined total body radiation) before the administration of therapeutic cells, so as to reduce endogenous immune cells in the patient, prevent the implanted exogenous therapeutic cells from being attacked, thereby reducing immune rejection reaction and creating a more favorable environment for the survival and expansion of the therapeutic cells in the patient. However, depletion of lymphocytes or bone marrow cells often leads to severe side effects. Therefore, it is necessary to reduce or eliminate the host's immune rejection reaction against the therapeutic cells to increase the persistence of the therapeutic cells in the body and enhance the therapeutic effect of ACT, while reducing the need for depleting lymphocyte or bone marrow cells to avoid generating associated side effects thereof.

To reduce or eliminate immune rejection reaction of the host against the therapeutic cells, current studies have used gene editing technology to knock out TCR genes or HLA class I molecules in therapeutic cells to reduce graft rejection caused by therapeutic cells. However, gene-edited cells still have immunogenicity and will trigger responses from other types of immune cells in the host. For example, studies show that knockout of B2M in donor T cells increases the risk of host NK cell-mediated graft rejection. Therefore, there is still a need to improve the existing cell therapies, particularly to reduce the immune rejection reaction of the patient.

### SUMMARY

Unless otherwise specified, all scientific and technical terms used herein have the same meaning as commonly understood by one of ordinary skill in the art to which the present invention pertains.

### I. Immunosuppressive molecules

In a first aspect, the present invention provides an immunosuppressive molecule comprising an immunosuppressive protein binding domain, a transmembrane domain and a co-stimulatory domain and the molecule does not comprise a primary signaling domain.

The term "immunosuppressive molecule" refers to a molecule capable of binding to an immunosuppressive protein (e.g. NKG2A, TIM3, LAG3, TIGIT, CTLA4, PD1, FasL) and in turn suppressing immune rejection of exogenous cells by a subject, for example, reducing the killing function of immune cells (e.g. T cells, NK cells, etc.) in the subject or suppressing the excessive proliferation of immune cells. The term "immunosuppressive protein" refers to a ligand or receptor that exert a suppressive effect on immune response, which plays an important role in maintaining autoimmune tolerance and regulating the duration and magnitude of immune response, thereby preventing the immune system from damaging and destroying normal tissues. The immunosuppressive molecule of the present invention binds to two or more of the immunosuppressive proteins NKG2A, TIM3, LAG3, TIGIT, CTLA4, PD1, and FasL. Preferably, the immunosuppressive molecule of the present invention binds to any one of the following combinations of immunosuppressive proteins: NKG2A and TIM3, NKG2A and LAG3, NKG2A and TIGIT, NKG2A and CTLA4, CTLA4 and TIM3, CTLA4 and LAG3, CTLA4 and TIGIT, PD1 and TIM3, PD1 and LAG3, PD1 and TIGIT, PD1 and CTLA4, TIM3 and LAG3, TIM3 and TIGIT, LAG3 and TIGIT, PD1 and NKG2A, FasL and NKG2A, FasL and LAG3, FasL and TIGIT, FasL and CTLA4, or FasL and PD1. More preferably, the immunosuppressive molecule of the present invention binds to any one of the following combinations of immunosuppressive proteins: NKG2A and TIM3, NKG2A and LAG3, NKG2A and TIGIT, NKG2A and CTLA4, CTLA4 and TIM3, CTLA4 and LAG3, CTLA4 and TIGIT, PD1 and TIM3, PD1 and LAG3, PD1 and TIGIT, PD1 and CTLA4, PD1 and NKG2A, FasL and PD1.

The term "immunosuppressive protein binding domain" refers to any structure (e.g. an antibody, a ligand, or a receptor, etc.) that can bind to an immunosuppressive protein or a functional variant thereof.

In some embodiments, the immunosuppressive protein binding domain of the present invention is selected from an antibody. The term "antibody" has the broadest meaning as understood by those skilled in the art and includes intact antibodies, such as monoclonal antibodies, polyclonal antibodies, multivalent antibodies, and multispecific antibodies (e.g., bispecific antibodies), and antibody fragments or synthetic polypeptides carrying one or more CDR sequences capable of exhibiting the desired biological activity, which may be of any class (e.g., IgG, IgE, IgM, IgD, IgA, etc.) or subclass (e.g., IgG1, IgG2, IgG2a, IgG3, IgG4, IgA1, IgA2, etc.). The term "antibody fragment" refers to at least a moiety of an intact antibody or variant thereof, and refers to a binding domain (e.g., the antigen variable region of an intact antibody) sufficient to confer upon the antibody fragment the ability to recognize and specifically bind to a target (e.g., an antigen). Examples of the antibody fragment include, but are not limited to, Fab, Fab', F(ab')2, Fd fragment, Fd', Fv fragment, scFv, disulfide-linked Fv (sdFv), linear antibody, "diabody" with two antigen binding sites, single domain antibody (sdAb) (e.g., heavy chain variable region VH, light chain variable region VL, nanobody VHH, etc. of an antibody).

In some embodiments, the immunosuppressive protein binding domain of the present invention is selected from a ligand, a receptor and a functional fragment thereof (i.e., a functional fragment with immunosuppressive protein binding ability, e.g. an extracellular region). In an embodiment, the three domains comprised in the immunosuppressive molecule, namely the immunosuppressive protein binding domain, the transmembrane domain and the co-stimulatory domain, are not all derived from the same molecule. For example, two of the domains are derived from the same molecule, while the other domain is derived from a different molecule, or all three domains are derived from different molecules. In other words, the immunosuppressive molecule of the present invention is not a naturally occurring full-length ligand or receptor itself. The term "ligand or receptor" refers to any molecule or atom capable of binding to an immunosuppressive protein and interacting with it. A ligand or receptor can be a naturally occurring molecule such as an organic or inorganic molecule, or a synthetic molecule. For example, the known ligands of NKG2A include HLA-E, the known ligands of TIM3 include Galectin9, HMGB1 and CEACAM1, the known ligands of LAG include Galectin3, LSECtin, FGL1 and certain MHC class II molecules, the known ligands of TIM include CD112, CD113, CD155 and Nectin4, the ligands of CTLA4 include CD80 and CD86, the known ligands of PD1 include PDL1 and PDL2, and the known receptors of FasL include Fas.

Unless the context clearly dictates otherwise, the "immunosuppressive protein binding domain" of the present invention encompasses the antibodies, ligands and functional fragments thereof as described above. Therefore, the immunosuppressive protein binding domain as described in the present invention is selected from the group consisting of an intact antibody, Fab, Fab', F(ab')2, an Fd fragment, Fd', an Fv fragment, scFv, sdFv, a linear antibody, a diabody, sdAbs, and a functional fragment of a ligand or a receptor.

The term "functional variant" or "functional fragment" refers to a variant or fragment that contains at least one amino acid modification (i.e., substitution, deletion, or insertion) compared to the parent amino acid sequence but retains the biological activity of the parent amino acid sequence. For example, the functional fragment of a ligand or receptor in the present invention generally refers to a fragment of a ligand or receptor capable of binding to the corresponding immunosuppressive protein, e.g. an extracellular region.

The term "heavy chain" refers to the larger of the two types of polypeptide chains present in a naturally occurring conformation in an antibody molecule and generally determines the class to which the antibody pertains. The term "light chain" refers to the smaller of the two types of polypeptide chains present in a naturally occurring conformation in an antibody molecule. Kappa (κ) and lambda (λ) light chains refer to the two major antibody light chain isotypes.

The term "complementarity determining region" or "CDR" refers to the amino acid sequence within the variable region of an antibody that confers antigen specificity and binding affinity. For example, generally, there are three CDRs present in each heavy chain variable region (e.g., CDR1-H, CDR2-H, and CDR3-H), and three CDRs in each light chain variable region (CDR1-L, CDR2-L, and CDR3-L). The precise amino acid sequence boundaries for a CDR can be determined using any of a number of well-known schemes, including: the Kabat numbering scheme, the Chothia numbering scheme, the IMGT numbering scheme, the AHo numbering scheme, and the AbM numbering scheme. The precise amino acid sequence for a given CDR or FR may vary depending on the numbering scheme chosen. It is understood that a "CDR" or "FR" of a given antibody or region thereof (e.g., a variable region thereof) encompasses CDRs or FRs as defined by any of the above or other known schemes. Where a designated CDR or FR contains a given amino acid sequence, it is understood that such CDR or FR may also have the sequence of the corresponding CDR or FR as defined by any of the above or other known schemes. The numbering scheme used herein to define the boundaries of CDRs and FRs is the Chothia scheme.

The term "single chain antibody" or "scFv" refers to a fusion protein comprising at least one light chain variable region (VL) and at least one heavy chain variable region (VH), wherein the light and heavy chain variable regions are contiguous (e.g., linked via a linker) and capable of being expressed as a single chain polypeptide, and wherein the scFv retains the specificity of the intact antibody from which it is derived. Unless otherwise specified, the scFv herein may have the VL and VH in any sequence (e.g., relative to the N-terminus and C-terminus of the polypeptide), and the scFv may contain either VL-linker-VH or VH-linker-VL from the N-terminus to the C-terminus. The term "linker" refers to a molecular sequence that links two molecules or two sequences within the same molecule. In some embodiments, the linker is a peptide linker. Preferably, the linker does not adversely affect the expression, secretion, or biological activity of the polypeptide. In addition, the linker is preferably not antigenic and does not cause immune response. In some embodiments, the linker may be an endogenous amino acid sequence, an exogenous amino acid sequence (e.g., a GS-rich sequence), or a non-peptide chemical linker. In an embodiment, the linker of the present invention has at least 70%, preferably at least 80%, more preferably at least 90%, 95%, 97% or 99%, or 100% sequence identity to the amino acid sequence as set forth in SEQ ID NO: 111 or 112.

The term "single domain antibody or "sdAb" refers to a single antigen binding polypeptide with three complementarity determining regions (CDRs), which includes full-length antibodies (e.g., HCAbs) and antigen binding fragments thereof (e.g., VH, VL, VHH). In some cases, the single domain antibody is selected from or engineered based on camelid- or shark-derived HCAb, and its heavy chain variable domain is referred to herein as "VHH". VHH has the following basic structure from N-terminus to C-terminus: FR1-CDR1-FR2-CDR2-FR3-CDR3-FR4, wherein FR1 to FR4 refer to framework regions 1 to 4, respectively, and CDR1 to CDR3 refer to complementarity determining regions 1 to 3.

In some embodiments, the immunosuppressive molecule comprises: (1) an immunosuppressive protein binding region comprising a first immunosuppressive protein binding domain and a second immunosuppressive protein binding domain; (2) a transmembrane domain; and (3) a co-stimulatory domain. In this embodiment, the first immunosuppressive protein binding domain and the second immunosuppressive protein binding domain are preferably linked by a linker as described above. In some other embodiments, the immunosuppressive molecule comprises: (1) a first unit structure targeting a first immunosuppressive protein, comprising a first immunosuppressive protein binding domain, a transmembrane domain, and a co-stimulatory domain; and (2) a second unit structure targeting a second immunosuppressive protein, comprising a second immunosuppressive protein binding domain, a transmembrane domain, and a co-stimulatory domain. In this embodiment, the first unit structure targeting the first immunosuppressive protein and the second unit structure targeting the second immunosuppressive protein are preferably linked by a self-cleaving peptide, e.g. as P2A, T2A, E2A, F2A, etc. Preferably, the first immunosuppressive protein and the second immunosuppressive protein of the present invention are selected from the following combinations consisting of: NKG2A and TIM3, NKG2A and LAG3, NKG2A and TIGIT, NKG2A and CTLA4, CTLA4 and TIM3, CTLA4 and LAG3, CTLA4 and TIGIT, PD1 and TIM3, PD1 and LAG3, PD1 and TIGIT, PD1 and CTLA4, TIM3 and LAG3, TIM3 and TIGIT, LAG3 and TIGIT, PD1 and NKG2A, FasL and NKG2A, FasL and LAG3, FasL and TIGIT, FasL and CTLA4, or FasL and PD1. More preferably, the first immunosuppressive protein and the second immunosuppressive protein of the present invention are selected from the following combinations consisting of: NKG2A and TIM3, NKG2A and LAG3, NKG2A and TIGIT, NKG2A and CTLA4, CTLA4 and TIM3, CTLA4 and LAG3, CTLA4 and TIGIT, PD1 and TIM3, PD1 and LAG3, PD1 and TIGIT, PD1 and CTLA4, PD1 and NKG2A, or FasL and PD1.

### NKG2A

In some embodiments, the immunosuppressive molecule of the present invention comprises an NKG2A binding domain, the NKG2A binding domain being an NKG2A-targeting antibody, a ligand, or functional fragment thereof. NKG2A, also known as KLRC1 or CD159A, is a member of the C-type lectin superfamily and a type II transmembrane protein primarily expressed in cytotoxic lymphocytes (e.g. CD8+ T cells and NK cells), comprising a cytoplasmic domain, a transmembrane domain, and an extracellular lectin-like domain. Its intracellular portion contains two immunoreceptor tyrosine-based inhibitory motifs (ITIMs), which are involved in inhibitory signal transduction. NKG2A is capable of forming a heterodimeric receptor with CD94, and NKG2A/CD94 transmits inhibitory signals to NK cells and CD8+ T cells, etc. The natural ligand for NKG2A is HLA-E, and the interaction between NKG2A and HLA-E contributes to tumor immune evasion, and disrupting the interaction between NKG2A and HLA-E has been demonstrated to effectively enhance anti-tumor immune response. Additionally, more and more study results show that NKG2A also plays an important role in other immune-related diseases, including viral infections, autoimmune diseases, inflammatory diseases, parasitic infections, etc.

In some embodiments, the NKG2A binding domain is an NKG2A-targeting antibody. Anti-NKG2A antibodies known in the art can all be used in the present invention. In some embodiments, the NKG2A-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 7; and the CDR1-L, CDR2-L and CDR3-L comprised in the light chain variable region are the same as the CDR1-L, CDR2-L and CDR3-L comprised in SEQ ID NO: 8. In some embodiments, CDR1-H comprised in the heavy chain variable region is as set forth in SEQ ID NO: 1, CDR2-H is as set forth in SEQ ID NO: 2, and CDR3-H is as set forth in SEQ ID NO: 3; CDR1-L comprised in the light chain variable region is as set forth in SEQ ID NO: 4, CDR2-L is as set forth in SEQ ID NO: 5, and CDR3-L is as set forth in SEQ ID NO: 6.

In some embodiments, the NKG2A-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 7; wherein the light chain variable region has at least 90%, at least 91%, at least 92%, at least 93%, at least 94%, at least 95%, at least 96%, at least 97%, at least 98%, at least 99%, or 100% identity to SEQ ID NO: 8. Preferably, the NKG2A-targeting antibody of the present invention comprises a heavy chain variable region as set forth in SEQ ID NO: 7 and a light chain variable region as set forth in SEQ ID NO:8.

In some embodiments, the NKG2A-targeting antibody has at least 90%, at least 91%, at least 92%, at least 93%, at least 94%, at least 95%, at least 96%, at least 97%, at least 98%, at least 99%, or 100% identity to SEQ ID NO: 9. Preferably, the NKG2A-targeting antibody is as set forth in SEQ ID NO: 9.

In some other embodiments, the NKG2A binding domain comprised in the immunosuppressive molecule of the present invention is an NKG2A-binding ligand, for example, HLA-E or a functional fragment thereof (i.e., a fragment having NKG2A binding function, for example the extracellular region of HLA-E). Preferably, the NKG2A-binding ligand comprises an amino acid having at least 90%, at least 91%, at least 92%, at least 93%, at least 94%, at least 95%, at least 96%, at least 97%, at least 98%, at least 99%, or 100% identity to SEQ ID NO: 10 or 11. More preferably, the NKG2A-binding ligand comprises the amino acid sequence as set forth in SEQ ID NO: 10 or 11. In this embodiment, the transmembrane region of the immunosuppressive molecule may be the transmembrane region of HLA-E. However, typically, the immunosuppressive molecule of this embodiment does not comprise the intracellular region of HLA-E. In other words, the immunosuppressive molecule is not full-length HLA-E.

### TIM3

In some embodiments, the immunosuppressive molecule of the present invention comprises a TIM3 binding domain, the TIM3 binding domain being a TIM3-targeting antibody, a ligand, or functional fragment thereof. TIM3, also known as HAVCR2 or CD366, is a member of the TIM family and a type I transmembrane protein expressed in a variety of immune cells (e.g. Th1 cells, Th17 cells, monocytes, dendritic cells, macrophages, etc.), comprising an extracellular immunoglobulin variable domain (IgV), a mucin domain, a transmembrane domain, and an intracellular domain. Studies have found that TIM3 binds to different ligands (e.g. Galectin9, HMGB1, CEACAM1), triggering signal transduction in immune cells. Galectin9 has high affinity for the TIM3 IgV domain, and studies have shown that the TIM3/Galectin9 signaling pathway is capable of mediating T cell exhaustion. In addition, Galectin9 can promote the maturation of TIM3+ antigen-presenting cells, thereby enhancing adaptive immunity. Therefore, the TIM3/Galectin9 signaling pathway plays an important role in organism immunomodulation. HMGB1 is a DNA binding protein that can function as a chromatin factor, and also plays an important role in inflammation, immune responses, DC differentiation, maintenance of genomic stability, and nuclear transcriptional regulation. HMGB1 acts as a ligand of TIM3, in the tumor microenvironment, tumor-infiltrating DCs highly expressing TIM3 protein competitively bind to HMGB1 against nucleic acids released by tumor cells, preventing nucleic acids from entering endosomes, thereby suppressing nucleic acid-stimulated innate immune response. Therefore, the activation and function of innate immune cells are disrupted, and blocking TIM3-mediated nucleic acid signaling may contribute to DNA vaccine research. CEACAM1, as a ligand for TIM3, binds to TIM3 through its N-terminal domain to form a cis-heterodimer, promoting stable expression of TIM3 on the surface of immune cells. However, trans-interaction between CEACAM1 and TIM3 is capable of suppressing the function of immune cells. Both cis- and trans-interactions between CEACAM1 and TIM3 could maintain the tolerance of immune cells. Study results have shown that combined blockade of the CEACAM1 and TIM3 pathways can enhance anti-tumor immune responses and improve tumor clearance rates in a patient with colorectal cancer. Therefore, the interaction between CEACAM1 and TIM3 plays a key role in regulating autoimmunity and anti-tumor immunity.

In some embodiments, the TIM3 binding domain is a TIM3-targeting antibody. Anti-TIM3 antibodies known in the art can all be used in the present invention. In some embodiments, the TIM3-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 19; and the CDR1-L, CDR2-L and CDR3-L comprised in the light chain variable region are the same as the CDR1-L, CDR2-L and CDR3-L comprised in SEQ ID NO: 20. In some embodiments, CDR1-H comprised in the heavy chain variable region is as set forth in SEQ ID NO: 13, CDR2-H is as set forth in SEQ ID NO: 14, and CDR3-H is as set forth in SEQ ID NO: 15; CDR1-L comprised in the light chain variable region is as set forth in SEQ ID NO: 16, CDR2-L is as set forth in SEQ ID NO: 17, and CDR3-L is as set forth in SEQ ID NO: 18.

In some embodiments, the TIM3-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 19; wherein the light chain variable region has at least 90%, at least 91%, at least 92%, at least 93%, at least 94%, at least 95%, at least 96%, at least 97%, at least 98%, at least 99%, or 100% identity to SEQ ID NO: 20. Preferably, the TIM3-targeting antibody of the present invention comprises a heavy chain variable region as set forth in SEQ ID NO: 19 and a light chain variable region as set forth in SEQ ID NO:20.

In some embodiments, the TIM3-targeting antibody has at least 90%, at least 91%, at least 92%, at least 93%, at least 94%, at least 95%, at least 96%, at least 97%, at least 98%, at least 99%, or 100% identity to SEQ ID NO: 21. Preferably, the TIM3-targeting antibody is as set forth in SEQ ID NO: 21.

In some other embodiments, the TIM3 binding domain comprised in the immunosuppressive molecule of the present invention is an TIM3-binding ligand, for example, Galectin9, HMGB1, CEACAM1 or a functional fragment thereof (i.e., a fragment having TIM3 binding function, for example the extracellular region of CEACAM1). Preferably, the TIM3-binding ligand comprises an amino acid sequence having at least 90%, at least 91%, at least 92%, at least 93%, at least 94%, at least 95%, at least 96%, at least 97%, at least 98%, at least 99%, or 100% identity to the amino acid sequence as set forth in any one of SEQ ID NOs: 22-24. More preferably, the TIM3-binding ligand comprises the amino acid sequence as set forth in any one of SEQ ID NOs: 22-24. In a specific embodiment, the TIM3-binding ligand comprises an extracellular region of CEACAM1 as set forth in SEQ ID NO: 24. In this embodiment, the transmembrane region of the immunosuppressive molecule is preferably the transmembrane region of CEACAM1. However, typically, the immunosuppressive molecule of this embodiment does not comprise the intracellular region of CEACAM1. In other words, the immunosuppressive molecule is not full-length CEACAM1.

### LAG3

In some embodiments, the immunosuppressive molecule of the present invention comprises an LAG3 binding domain, the LAG3 binding domain being an LAG3-targeting antibody, a ligand, or functional fragment thereof. LAG3, also known as CD223, is a type I transmembrane protein, pertaining to the immunoglobulin superfamily (IgSF). It is expressed as dimers or oligomers on the surfaces of activated CD4+ and CD8+ T cells, regulatory T cells, Tr1 cells, NK cells, and plasmacytoid dendritic cells (pDCs). The LAG3 protein shares a homologous structure with CD4, with both having four Ig-like domains in the extracellular regions. However, LAG3 contains an additional looped structure within the membrane-distal Ig-like domain, which is absent in CD4. This extra looped structure enables LAG3 protein to bind to MHC class II molecules (e.g. HLA-DR, HLA-DP, and HLA-DQ antigens, etc.) expressed on antigen-presenting cells (APCs) or tumor cells, which has significantly higher affinity than CD4, and can negatively regulate T cell receptor (TCR) signaling. In addition to MHC class II molecules, Galectin-3, LSECtin, and FGL1 are also LAG3 ligands. These three proteins are expressed on various tumor cells and suppress T cell response. Studies have shown that LAG3 can negatively regulate T cell activation, proliferation, and cytokine production, suppress pDC activation, and enhance the suppressive activity of regulatory T cells.

In some embodiments, the LAG3 binding domain is a TIM3-targeting antibody. Anti-LAG3 antibodies known in the art can all be used in the present invention. In some embodiments, the LAG3-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 32; and the CDR1-L, CDR2-L and CDR3-L comprised in the light chain variable region are the same as the CDR1-L, CDR2-L and CDR3-L comprised in SEQ ID NO: 33. In some embodiments, CDR1-H comprised in the heavy chain variable region is as set forth in SEQ ID NO: 26, CDR2-H is as set forth in SEQ ID NO: 27, and CDR3-H is as set forth in SEQ ID NO: 28; CDR1-L comprised in the light chain variable region is as set forth in SEQ ID NO: 29, CDR2-L is as set forth in SEQ ID NO: 30, and CDR3-L is as set forth in SEQ ID NO: 31.

In some embodiments, the LAG3-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 32; wherein the light chain variable region has at least 90%, at least 91%, at least 92%, at least 93%, at least 94%, at least 95%, at least 96%, at least 97%, at least 98%, at least 99%, or 100% identity to SEQ ID NO: 33. Preferably, the LAG3-targeting antibody of the present invention comprises a heavy chain variable region as set forth in SEQ ID NO: 32 and a light chain variable region as set forth in SEQ ID NO:33.

In some embodiments, the LAG3-targeting antibody has at least 90%, at least 91%, at least 92%, at least 93%, at least 94%, at least 95%, at least 96%, at least 97%, at least 98%, at least 99%, or 100% identity to SEQ ID NO: 34. Preferably, the LAG3-targeting antibody is as set forth in SEQ ID NO: 34.

In some other embodiments, the LAG3 binding domain comprised in the immunosuppressive molecule of the present invention is an LAG3-binding ligand, for example, an MHC class II molecule, Galectin 3, LSECtin, FGL1, or a functional fragment thereof (i.e., a fragment having LAG3 binding function, for example the extracellular region of LSECtin). Preferably, the LAG3-binding ligand comprises an amino acid sequence having at least 90%, at least 91%, at least 92%, at least 93%, at least 94%, at least 95%, at least 96%, at least 97%, at least 98%, at least 99%, or 100% identity to the amino acid sequence as set forth in any one of SEQ ID NOs: 35-37. More preferably, the LAG3-binding ligand comprises the amino acid sequence as set forth in any one of SEQ ID NOs: 35-37. In a specific embodiment, the LAG3-binding ligand comprises an extracellular region of LSECtin as set forth in SEQ ID NO: 37. In this embodiment, the transmembrane region of the immunosuppressive molecule is preferably the transmembrane region of LSECtin. However, typically, the immunosuppressive molecule of this embodiment does not comprise the intracellular region of LSECtin. In other words, the immunosuppressive molecule is not full-length LSECtin.

### TIGIT

In some embodiments, the immunosuppressive molecule of the present invention comprises a TIGIT binding domain, the TIGIT binding domain being a TIGIT-targeting antibody, a ligand, or functional fragment thereof. TIGIT, also known as VSTM3, VSIG9, or WUCAM, is a member of the PVR-like protein family, and a type I transmembrane protein primarily expressed in NK cells and T cells (e.g. CD4+ T cells, CD8+ T cells, and Treg cells, etc.), comprising an extracellular immunoglobulin variable domain, a transmembrane domain, and a short intracellular domain, which having an immunoreceptor tyrosine-based inhibitory motif (ITIM) and an immunoglobulin tyrosine-based tail (ITT)-like motif. TIGIT has four ligands: CD112, CD113, CD155, and Nectin4, which all pertain to the Nectin/NECL family, the family participating in mediating cell adhesion, cell polarization, and other processes. TIGIT binds to CD155 and Nectin4 with significantly higher affinity than CD112 and CD113. At the same time, CD155 also functions as a ligand for the activating receptor CD226. When CD155 binds to CD226, it transmits an activatiing signal to immune cells. When CD155 binds to TIGIT, it recruits the SHP1 phosphatase to the membrane through its ITIM motif to transmit a inhibitory signal, subsequently inactivating numerous proteins involved in T cell effector functions. Nectin4 is the only member of the Nectin/NECL family of proteins that could specifically bind to TIGIT without interacting with CD226, CD96, or CD112R.

In some embodiments, the TIGIT binding domain is a TIGIT-targeting antibody. Anti-TIGIT antibodies known in the art can all be used in the present invention. In some embodiments, the TIGIT-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 45; and the CDR1-L, CDR2-L and CDR3-L comprised in the light chain variable region are the same as the CDR1-L, CDR2-L and CDR3-L comprised in SEQ ID NO: 46. In some embodiments, CDR1-L comprised in the light chain variable region is as set forth in SEQ ID NO: 39, CDR2-L is as set forth in SEQ ID NO: 40, and CDR3-L is as set forth in SEQ ID NO: 41; CDR1-H comprised in the heavy chain variable region is as set forth in SEQ ID NO: 42, CDR2-H is as set forth in SEQ ID NO: 43, and CDR3-H is as set forth in SEQ ID NO: 44.

In some embodiments, the TIGIT-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 45; wherein the light chain variable region has at least 90%, at least 91%, at least 92%, at least 93%, at least 94%, at least 95%, at least 96%, at least 97%, at least 98%, at least 99%, or 100% identity to SEQ ID NO: 46. Preferably, the TIGIT-targeting antibody of the present invention comprises a heavy chain variable region as set forth in SEQ ID NO: 45 and a light chain variable region as set forth in SEQ ID NO:46.

In some embodiments, the TIGIT-targeting antibody has at least 90%, at least 91%, at least 92%, at least 93%, at least 94%, at least 95%, at least 96%, at least 97%, at least 98%, at least 99%, or 100% identity to SEQ ID NO: 47. Preferably, the TIGIT-targeting antibody is as set forth in SEQ ID NO: 47.

In some other embodiments, the TIGIT binding domain comprised in the immunosuppressive molecule of the present invention is an TIGIT-binding ligand, for example, CD155, CD112, CD113, Nectin4, or a functional fragment thereof (i.e., a fragment having TIGIT binding function, for example the extracellular region of CD155, CD112, CD113, or Nectin4). Preferably, the TIGIT-binding ligand comprises an amino acid sequence having at least 90%, at least 91%, at least 92%, at least 93%, at least 94%, at least 95%, at least 96%, at least 97%, at least 98%, at least 99%, or 100% identity to the amino acid sequence as set forth in any one of SEQ ID NOs: 48-51. More preferably, the TIGIT-binding ligand comprises the amino acid sequence as set forth in any one of SEQ ID NOs: 48-51. In this embodiment, the transmembrane region of the immunosuppressive molecule can be the corresponding transmembrane region of CD155, CD112, CD113, or Nectin4. However, typically, the immunosuppressive molecule of this embodiment does not comprise the corresponding intracellular region of CD155, CD112, CD113, or Nectin 4. In other words, the immunosuppressive molecule is not full-length CD155, CD112, CD113 or Nectin 4.

### CTLA4

In some embodiments, the immunosuppressive molecule of the present invention comprises a CTLA4 binding domain, the CTLA4 binding domain being a CTLA4-targeting antibody, a ligand, or functional fragment thereof. CTLA4, also known as CD152 or CELIAC3, is a member of the CD28 immunoglobulin subfamily and a type I transmembrane protein primarily expressed on T cells, comprising an extracellular immunoglobulin variable domain, a transmembrane domain, and a conserved cytoplasmic tail region. The extracellular domain of the CD28 immunoglobulin subfamily contains a highly conserved motif (MYPPPY), which is crucial for binding its ligands CD80 and CD86. Ligands for CTLA4, CD80 and CD86, are typically found on the surface of antigen-presenting cells and can bind CD28 or CTLA4, respectively, to generate costimulatory or cosuppressive responses, but CD80 and CD86 bind CTLA4 with higher affinity. Due to this suppressive effect, CTLA4 is a key regulator for regulating T cell homeostasis and self-tolerance.

In some embodiments, the CTLA4 binding domain is a CTLA4-targeting antibody. Anti-CTLA4 antibodies known in the art can all be used in the present invention. In some embodiments, the CTLA4-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 62; and the CDR1-L, CDR2-L and CDR3-L comprised in the light chain variable region are the same as the CDR1-L, CDR2-L and CDR3-L comprised in SEQ ID NO: 63. In some embodiments, CDR1-H comprised in the heavy chain variable region is as set forth in SEQ ID NO: 56, CDR2-H is as set forth in SEQ ID NO: 57, and CDR3-H is as set forth in SEQ ID NO: 58; CDR1-L comprised in the light chain variable region is as set forth in SEQ ID NO: 59, CDR2-L is as set forth in SEQ ID NO: 60, and CDR3-L is as set forth in SEQ ID NO: 61.

In some embodiments, the CTLA4-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 62; wherein the light chain variable region has at least 90%, at least 91%, at least 92%, at least 93%, at least 94%, at least 95%, at least 96%, at least 97%, at least 98%, at least 99%, or 100% identity to SEQ ID NO: 63. Preferably, the CTLA4-targeting antibody of the present invention comprises a heavy chain variable region as set forth in SEQ ID NO: 62 and a light chain variable region as set forth in SEQ ID NO:63.

In some embodiments, the CTLA4-targeting antibody has at least 90%, at least 91%, at least 92%, at least 93%, at least 94%, at least 95%, at least 96%, at least 97%, at least 98%, at least 99%, or 100% identity to SEQ ID NO: 64. Preferably, the CTLA4-targeting antibody is as set forth in SEQ ID NO: 64.

In some other embodiments, the CTLA4 binding domain comprised in the immunosuppressive molecule of the present invention is an CTLA4-binding ligand, for example, CD80, CD86 or a functional fragment thereof (i.e., a fragment having CTLA4 binding function, for example the extracellular region of CD80 or CD86). Preferably, the CTLA4-binding ligand comprises an amino acid sequence having at least 90%, at least 91%, at least 92%, at least 93%, at least 94%, at least 95%, at least 96%, at least 97%, at least 98%, at least 99%, or 100% identity to SEQ ID NO: 65 or 66. More preferably, the CTLA4-binding ligand comprises the amino acid sequence as set forth in SEQ ID NO: 65 or 66. In this embodiment, the transmembrane region of the immunosuppressive molecule is preferably the transmembrane region of CD80 or CD86. However, typically, the immunosuppressive molecule of this embodiment does not comprise the intracellular region of CD80 or CD86. In other words, the immunosuppressive molecule is not full-length CD80 or CD86.

### PD1

In some embodiments, the immunosuppressive molecule of the present invention comprises a PD1 binding domain, the PD1 binding domain being a PD1-targeting antibody, a ligand, or functional fragment thereof. PD1, also known as CD279, is a member of the immunoglobulin superfamily and a type I transmembrane protein primarily expressed on activated T cells, NK cells, B cells, macrophages, dendritic cells, and monocytes, comprising an extracellular immunoglobulin variable domain, a transmembrane domain, and a cytoplasmic tail domain.

The cytoplasmic tail domain thereof contains two tyrosine-based motifs: an immunoreceptor tyrosine-based inhibition motif (ITIM) and an immunoreceptor inhibitory tyrosine-based switch motif (ITSM). Studies have shown that the ITSM is necessary for PD1 to exert the immunosuppressive function on activated T cells. Ligands for PD1 include PDL1 (B7-H1, CD274) and PDL2 (B7-DC, CD273), both of which pertain to the protein B7 family. PD1 exerts negative immune regulatory effect through interaction with its ligands, PDL1 and PDL2. When PD1 interacts with its ligands, its intracellular ITSM becomes phosphorylated and recruits the corresponding phosphatases SHP-1 and SHP-2, leading to dephosphorylation of downstream signaling molecules and thus downregulating immune cell response levels. This negative regulatory mechanism of the immune system is a key molecular basis for maintaining immune tolerance. Numerous studies have shown that the immunosuppressive state of an organism induced by the overexpression of negative immune regulatory molecules such as PD1 and its interaction with the receptor PD-L1/PD-L2 plays an important role in the pathogenesis of cancer and chronic infectious diseases such as HIV, HCV, and HBV.

In some embodiments, the PD1 binding domain is a PD1-targeting antibody. Anti-PD1 antibodies known in the art can all be used in the present invention. In some embodiments, the PD1-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 75; and the CDR1-L, CDR2-L and CDR3-L comprised in the light chain variable region are the same as the CDR1-L, CDR2-L and CDR3-L comprised in SEQ ID NO: 76. In some embodiments, CDR1-H comprised in the heavy chain variable region is as set forth in SEQ ID NO: 69, CDR2-H is as set forth in SEQ ID NO: 70, and CDR3-H is as set forth in SEQ ID NO: 71; CDR1-L comprised in the light chain variable region is as set forth in SEQ ID NO: 72, CDR2-L is as set forth in SEQ ID NO: 73, and CDR3-L is as set forth in SEQ ID NO: 74.

In some embodiments, the PD1-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 75; wherein the light chain variable region has at least 90%, at least 91%, at least 92%, at least 93%, at least 94%, at least 95%, at least 96%, at least 97%, at least 98%, at least 99%, or 100% identity to SEQ ID NO: 76. Preferably, the PD1-targeting antibody of the present invention comprises a heavy chain variable region as set forth in SEQ ID NO: 75 and a light chain variable region as set forth in SEQ ID NO:76.

In some embodiments, the PD1-targeting antibody has at least 90%, at least 91%, at least 92%, at least 93%, at least 94%, at least 95%, at least 96%, at least 97%, at least 98%, at least 99%, or 100% identity to SEQ ID NO: 77. Preferably, the PD1-targeting antibody is as set forth in SEQ ID NO: 77.

In some other embodiments, the PD1 binding domain comprised in the immunosuppressive molecule of the present invention is a PD1-binding ligand, for example, PDL1, PDL2 or a functional fragment thereof (i.e., a fragment having PD1 binding function, for example the extracellular region of PDL1 or PDL2). Preferably, the PD1-binding ligand comprises an amino acid sequence having at least 90%, at least 91%, at least 92%, at least 93%, at least 94%, at least 95%, at least 96%, at least 97%, at least 98%, at least 99%, or 100% identity to SEQ ID NO: 78 or 79. More preferably, the PD1-binding ligand comprises the amino acid sequence as set forth in SEQ ID NO: 78 or 79. In this embodiment, the transmembrane region of the immunosuppressive molecule is preferably the transmembrane region of PDL1 or PDL2. However, typically, the immunosuppressive molecule of this embodiment does not comprise the intracellular region of PDL1 or PDL2. In other words, the immunosuppressive molecule is not full-length PDL1 or PDL2.

### FasL

In some embodiments, the immunosuppressive molecule of the present invention comprises an FasL binding domain, the FasL binding domain being an FasL-targeting antibody, a ligand, or functional fragment thereof. FasL is a type II cell membrane surface glycoprotein, pertaining to a member of the tumor necrosis factor receptor superfamily (TNFRSF). FasL is distributed on the surface of activated T cells, NK cells, mononuclear macrophages, tec., and its receptor is Fas. It has been found that the Fas/FasL system is one of the important pathways of cell apoptosis. Specifically, the binding of FasL to Fas will form a death-inducing complex, which in turn activates the caspase signaling pathway, and ultimately leads to cell apoptosis through the phosphorylation of tyrosine and serine in the cells. It is reported that the dysfunction of Fas/FasL is closely related to various clinical diseases, for example, autoimmune diseases (rheumatoid arthritis, systemic lupus erythematosus, etc.), acute immune hyperactivity (caused by organ transplantation, multiple trauma, etc.), chronic immune hyperactivity (Kawasaki's disease, idiopathic pulmonary fibrosis, etc.), chronic infection (EBV infection, inflammation caused by viruses, etc.), lymphoid tumors (T-ALL, T cell lymphomas, etc.), etc.

In some embodiments, the FasL binding domain is a FasL-targeting antibody. Anti-FasL antibodies known in the art can all be used in the present invention. In some embodiments, the FasL-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 119 or 125; and the CDR1-L, CDR2-L and CDR3-L comprised in the light chain variable region are the same as the CDR1-L, CDR2-L and CDR3-L comprised in SEQ ID NO: 120. In some embodiments, CDR1-H comprised in the heavy chain variable region is as set forth in SEQ ID NO: 113 or 122, CDR2-H is as set forth in SEQ ID NO: 114 or 123, and CDR3-H is as set forth in SEQ ID NO: 115 or 124; CDR1-L comprised in the light chain variable region is as set forth in SEQ ID NO: 116, CDR2-L is as set forth in SEQ ID NO: 117, and CDR3-L is as set forth in SEQ ID NO: 118.

In some embodiments, the FasL-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 119 or 125, and the light chain variable region has at least 90%, at least 91%, at least 92%, at least 93%, at least 94%, at least 95%, at least 96%, at least 97%, at least 98%, at least 99%, or 100% identity to SEQ ID NO: 120. Preferably, the FasL-targeting antibody of the present invention comprises a heavy chain variable region as set forth in SEQ ID NO: 119 or 125 and a light chain variable region as set forth in SEQ ID NO:120.

In some embodiments, the FasL-targeting antibody has at least 90%, at least 91%, at least 92%, at least 93%, at least 94%, at least 95%, at least 96%, at least 97%, at least 98%, at least 99%, or 100% identity to SEQ ID NO: 121 or 126. Preferably, the FasL-targeting antibody is as set forth in SEQ ID NO: 121 or 126.

In some other embodiments, the FasL binding domain comprised in the immunosuppressive molecule of the present invention is a FasL-binding receptor, for example, Fas or a functional fragment thereof (i.e., a fragment having Fas binding function, for example the extracellular domain of Fas). Preferably, the FasL-binding receptor comprises an amino acid sequence having at least 90%, at least 91%, at least 92%, at least 93%, at least 94%, at least 95%, at least 96%, at least 97%, at least 98%, at least 99%, or 100% identity to SEQ ID NO: 127. More preferably, the FasL-binding receptor comprises the amino acid sequence as set forth in SEQ ID NO: 127. In this embodiment, the transmembrane region of the immunosuppressive molecule is preferably the transmembrane region of Fas. However, typically, the immunosuppressive molecule of this embodiment does not comprise the intracellular region of Fas. In other words, the immunosuppressive molecule is not full-length Fas.

The term sequence "identity" means the degree to which two (nucleotide or amino acid) sequences in alignment have the same residue at the same position, and is usually expressed as a percentage. Preferably, identity is determined over the entire length of the sequences being compared. Therefore, two copies of the exact same sequence have 100% identity. Those skilled in the art know that several algorithms can be used to determine sequence identity, for example, Blast (Altschul et al. (1997) Nucleic Acids Res. 25:3389-3402), Blast2 (Altschul et al. (1990) J. Mol. Biol. 215: 403-410), Smith-Waterman (Smith et al. (1981) J. Mol. Biol. 147:195-197) and Clustal W.

The term "transmembrane domain" refers to a polypeptide structure that enables the expression of an immunosuppressive molecule on the cell surface, and anchors the immunosuppressive protein binding domain to the cell membrane. The transmembrane domain may be natural or synthetic, and also may be derived from any membrane-bound protein or transmembrane protein. The transmembrane domain is capable of signaling when a target binding domain binds to a target. The transmembrane domain particularly suitable for the present invention can be derived from, for example, a TCR α chain, a TCR β chain, a TCR γ chain, a TCR δ chain, a CD3 ζ subunit, a CD3 ε subunit, a CD3 γ subunit, a CD3 δ subunit, CD28, CD45, CD4, CD5, CD8α, CD9, CD16, CD22, CD33, CD37, CD47, CD64, CD80, CD86, CD94, CD112, CD113, CD134, CD137, CD154, CD155, KIRDS2, OX40, CD2, CD27, CD18, ICOS, 4-1BB, GITR, CD40, BAFFR, HVEM, SLAMF7, NKp80, CD160, BCMA, IL-2R β, IL-2R γ, IL-7R a, ITGA1, VLA1, CD49a, ITGA4, IA4, CD49D, ITGA6, VLA-6, CD49f, ITGAD, CD1 1d, ITGAE, CD103, ITGAL, CD1 1a, ITGAM, CD1 1b, ITGAX, CD1 1c, ITGB1, CD29, ITGB2, CD18, LFA-1, ITGB7, TNFR2, DNAM1, SLAMF4, CD84, CD96, HLA-E, LSECtin, PDL1, PDL2, CEACAM1, Fas, MHC class II molecules, CRT AM, Ly9, CD160, PSGL1, CDIOO, SLAMF6, SLAMF1, SLAMF8, CD162, LTBR, PAG/Cbp, NKp44, NKp30, NKp46, NKG2D or NKG2C. In some embodiments, the transmembrane domain is derived from the following molecules: CD8α, CD4, CD28 or 4-1BB, or the transmembrane domain may be synthetic and may mainly comprise hydrophobic residues such as leucine and valine. Preferably, the transmembrane domain is derived from CD28, which has at least 70%, preferably at least 80%, more preferably at least 90%, 95%, 97%, or 99%, or 100% sequence identity to the amino acid sequence as set forth in SEQ ID NO: 82, or the transmembrane domain is derived from CD8α, which has at least 70%, preferably at least 80%, more preferably at least 90%, 95%, 97%, or 99%, or 100% sequence identity to the amino acid sequence as set forth in SEQ ID NO: 83 or 84. In some other embodiments, the transmembrane domain comprised in the immunosuppressive molecule of the present invention is derived from a ligand or receptor targeting an immunosuppressive protein, for example HLA-E, CEACAM1, LSECtin, CD112, CD113, CD155, Nectin4, CD80, CD86, PDL1, PDL2, Fas and some MHC class II molecules, etc. Preferably, the transmembrane domain has at least 70%, preferably at least 80%, more preferably at least 90%, 95%, 97%, or 99%, or 100% sequence identity to the amino acid sequence as set forth in any one of SEQ ID NOs: 12, 25, 38, 52, 53, 54, 55, 67, 68, 80, 81, and 128. More preferably, the transmembrane domain is linked to the extracellular domain of the corresponding ligand or receptor, preferably directly linked.

In some embodiments, the immunosuppressive molecule further comprises a hinge region between the immunosuppressive protein binding domain and the transmembrane domain. The term "hinge region" generally refers to any oligopeptide or polypeptide that functions to link a transmembrane domain to an antibody. Specifically, the hinge region serves to provide greater flexibility and accessibility to the antibody. The hinge region may comprise up to 300 amino acids, preferably 10 to 100 amino acids and most preferably 25 to 50 amino acids. The hinge region may be completely or partially derived from a natural molecule, e.g., completely or partially from the extracellular region of CD8, CD4 or CD28, or completely or partially from an antibody constant region. Alternatively, the hinge region may be a synthetic sequence corresponding to a naturally occurring hinge sequence, or may be a completely synthetic hinge sequence. Preferably, the hinge region comprises a hinge region moiety of CD8α, CD28, an FcγRIIIα receptor, IgG4 or IgG1, more preferably a hinge from CD8α, CD28 or IgG4. In some embodiments, the hinge region is from CD28, which has at least 70%, preferably at least 80%, more preferably at least 90%, 95%, 97%, or 99%, or 100% sequence identity to the amino acid sequence as set forth in SEQ ID NO: 85. In some embodiments, the hinge region is from CD8α, which has at least 70%, preferably at least 80%, more preferably at least 90%, 95%, 97%, or 99%, or 100% sequence identity to the amino acid sequence as set forth in SEQ ID NO: 86 or 87. In some embodiments, the hinge region is from IgG4, which has at least 70%, preferably at least 80%, more preferably at least 90%, 95%, 97%, or 99%, or 100% sequence identity to the amino acid sequence as set forth in SEQ ID NO: 88.

The term "co-stimulatory domain" refers to at least a moiety of a protein that mediates intracellular signal transduction to induce immune response such as an effector function, which is an intracellular functional signaling domain from a co-stimulatory molecule, comprising the entire intracellular region of the co-stimulatory molecule, or a functional fragment thereof. A "co-stimulatory molecule" refers to a cognate binding partner that specifically binds to a co-stimulatory ligand, thereby mediating a co-stimulatory response (for example, proliferation and survival). The co-stimulatory signaling domain of any co-stimulatory molecule is suitable for the immunosuppressive molecule as described herein. The co-stimulatory molecule includes, but is not limited to, MHC class I molecules, BTLA, and Toll ligand receptors. The co-stimulatory domain of the present invention includes, but is not limited to, an intracellular region derived from the following proteins: LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, 4-1BB, CD270, CD272, B7-H3, ICOS, CD357, DAP10, DAP12, LAT, NKG2C, SLP76, PD1, LIGHT, TRIM, ZAP70 and a combination thereof. Preferably, the co-stimulatory domain of the CAR of the present invention is 4-1BB and/or CD28. In some embodiments, the co-stimulatory domain is from CD28, which has at least 70%, preferably at least 80%, more preferably at least 90%, 95%, 97%, or 99%, or 100% sequence identity to the amino acid sequence as set forth in SEQ ID NO: 89. In some embodiments, the co-stimulatory domain is from 4-1BB, which has at least 70%, preferably at least 80%, more preferably at least 90%, 95%, 97%, or 99%, or 100% sequence identity to the amino acid sequence as set forth in SEQ ID NO: 90 or 91.

The immunosuppressive molecule of the present invention does not comprise a primary signaling domain. The term "primary signaling domain" refers to a protein structure that acts together after antigen-receptor binding to trigger primary signaling, which is generally an intracellular sequence of a T cell receptor and a co-receptor. The primary signaling domain generally comprises one or more Immunoreceptor Tyrosine-based Activation Motifs (ITAM). The primary signaling domain of the present invention includes, but is not limited to, an intracellular region derived from the following proteins: FcRγ, FcRβ, CD3γ, CD3δ, CD3ε, CD3ζ, CD5, CD22, CD79a, CD79b, NFAM1, STAM1, STAM2, and CD66d. In some embodiments, the immunosuppressive molecule of the present invention does not comprise a CD3ζ intracellular region, for example, a CD3ζ intracellular region having at least 70%, preferably at least 80%, more preferably at least 90%, 95%, 97%, or 99%, or 100% sequence identity to the amino acid sequence as set forth in SEQ ID NO: 92 or 93.

In some embodiments, the immunosuppressive molecule of the present invention further comprises a signal peptide such that when it is expressed in a cell, e.g. a T cell, the nascent protein is directed to the endoplasmic reticulum and subsequently to the cell surface. The core of the signal peptide may contain a long hydrophobic amino acid segment, which has a tendency to form a single α-helix. At the end of the signal peptide, there is usually an amino acid segment recognized and cleaved by a signal peptidase. The signal peptidase may cleave during or after translocation, so as to generate a free signal peptide and a mature protein. Then, the free signal peptide is digested by a specific protease. The signal peptides that may be used in the present invention is well known to those skilled in the art, for example, the signal peptide derived from B2M, CD8α, IgG1, GM-CSFRα, etc. In some embodiments, the signal peptide that can be used in the present invention is from B2M, which has at least 70%, preferably at least 80%, more preferably at least 90%, 95%, 97%, or 99%, or 100% sequence identity to the amino acid sequence as set forth in SEQ ID NO: 94. In some embodiments, the signal peptide that can be used in the present invention is from CD8α, which has at least 70%, preferably at least 80%, more preferably at least 90%, 95%, 97%, or 99%, or 100% sequence identity to the amino acid sequence as set forth in SEQ ID NO: 95 or 96.

### II. Nucleic acids and vectors

The present invention further provides a nucleic acid molecule that comprises a nucleic acid sequence encoding the immunosuppressive molecule of the present invention. Optionally, the nucleic acid molecule may further comprise a nucleic acid sequence encoding a functional exogenous receptor (for example, chimeric antigen receptor, T cell receptor, etc.).

As used herein, the term "nucleic acid molecule" includes sequences of ribonucleotides and deoxyribonucleotides, e.g., modified or unmodified RNA or DNA, each in single- and/or double-stranded form, linear or circular, or mixtures thereof (including hybrid). Therefore, the nucleic acid according to the present invention includes DNA (e.g., dsDNA, ssDNA, cDNA), RNA (e.g., dsRNA, ssRNA, mRNA, ivtRNA), and combinations or derivatives thereof (e.g. PNAs). Preferably, the nucleic acid is DNA or RNA.

A nucleic acid may comprise conventional phosphodiester bonds or unconventional bonds (e.g., amide bonds, e.g., those found in peptide nucleic acids (PNAs)). The nucleic acid of the present invention may further comprise one or more modified bases, e.g., for example tritylated bases and unusual bases (e.g. inosine). Other modifications are also contemplated, including chemical, enzymatic or metabolic modifications, so long as the multi-chain CAR of the present invention can be expressed from the polynucleotide. The nucleic acid may be provided in isolated form. In some embodiments, the nucleic acid may further comprise regulatory sequences, e.g., transcriptional control elements (including promoters, enhancers, operators, repressors, and transcriptional termination signals) ribosome binding sites, introns, etc.

The nucleic acid sequences of the present invention may be codon-optimized for optimal expression in desired cells (e.g., immune cells); or for expression in bacteria, yeast or insect cells. Codon optimization refers to the replacement of codons in the target sequence that are generally rare in highly expressed genes of a given species with codons that are generally common in highly expressed genes of this species, while the codons before and after the replacement code for the same amino acid. Therefore, the choice of optimal codons depends on the codon usage bias of the host genome.

The present invention further provides a vector comprising the nucleic acid molecule of the present invention. Optionally, the nucleic acid sequence encoding the immunosuppressive molecule and the nucleic acid sequence encoding the functional exogenous receptor (for example, chimeric antigen receptor, chimeric T cell receptor, etc.) can be located on the same vector or on different vectors.

As used herein, the term "vector" is an intermediary nucleic acid molecule used to transfer (exogenous) genetic material into a host cell, and in the host cell the nucleic acid molecule can be, for example, replicated and/or expressed. Suitable vectors that can be used in the present invention are known in the art, and many are commercially available. In some embodiments, the vector of the present invention includes, but is not limited to, plasmids, viruses (for example, retrovirus, adenovirus, adeno-associated virus, vaccinia virus, vesicular stomatitis virus, measles virus, mumps virus, poliovirus, orthomyxovirus, parvovirus, Maraba virus, coxsackie virus, herpes virus, and lentivirus, etc.), phage, phagemid, cosmid, and artificial chromosome (including BAC and YAC). The vector typically also comprises an origin of autonomous replication in the cell (if stable expression of polynucleotide is desired), a selectable marker, and a restriction enzyme cleavage site (e.g., a multiple cloning site, MCS). The vector may additionally comprise elements such as a promoter, a poly-A tail (polyA), 3' UTR, an enhancer, a terminator, an insulator, an operon, a selectable marker, a reporter gene, a targeting sequence, and/or a protein purification tag. In a specific embodiment, the vector is an in vitro transcription vector.

### III. Engineered cells

The present invention further provides an engineered cell that expresses the immunosuppressive molecule, the nucleic acid encoding the immunosuppressive molecule, or the vector comprising the nucleic acid of the present invention. In some embodiments, the engineered cell of the present invention further expresses a functional exogenous receptor (as defined below).

### Cells

In some embodiments, the engineered cell of the present invention is an engineered immune cell.

The term "immune cell" refers to any cell of the immune system that has one or more effector functions (e.g., cytotoxic cell killing activity, secretion of cytokines, induction of ADCC and/or CDC). For example, the immune cell may be a B cell, a T cell, a macrophage, a dendritic cell, a monocyte, an NK cell, or an NKT cell. The immune cell may be obtained from a variety of sources, for example from a subject (e.g., isolated from peripheral blood monocytes, bone marrow, lymph node tissue, cord blood, thymus tissue, tissue from sites of infection, ascites, pleural effusion, spleen tissue, tumors, etc. of the subject), or from a cell line cultured in vitro (e.g., Jurkat, SupT1, NK92, etc.), or differentiated from stem cells (e.g., derived from cord blood stem cells, progenitor cells, bone marrow stem cells, hematopoietic stem cells, adult stem cells, embryonic stem cells, pluripotent stem cells, iPSCs, etc.). Preferably, the immune cell is a T cell or an NK cell, more preferably a T cell. The T cell also may be concentrated or purified. T cells may be at any developmental stage, including but not limited to: a CD4+CD8+ T cell, a CD4+ T cell (for example, Th1 and Th2 cells), CD8+ T cell (for example, cytotoxic T cell), a CD4-CD8- T cell, a tumor infiltrating cell, a memory T cell, a naive T cell, a γδ-T cell, an αβ-T cell, etc. Preferably, the immune cell is a human T cell, which can be obtained using a variety of techniques known to those skilled in the art, such as isolating T cells from the blood of a subject through Ficoll.

In some other embodiments, the cell is a stem cell.

The term "stem cell" refers to a primitive cell with the potential for self-replication, multi-lineage differentiation and homing. It is the origin cell of an organism, and the ancestral cell that gives rise to various tissues and organs of the human body. During the process of cell differentiation, the uneven distribution of regulatory differentiation proteins in the cytoplasm causes one daughter cell irreversibly proceeds toward terminal differentiation, becoming a differentiated cell with specific function until it completely loses the ability to divide again, eventually leading to cellular senescence and death. To compensate for this deficiency, the organism retains a portion of undifferentiated primitive cells, which preserve the parental characteristics. This portion of the retained undifferentiated primitive cells is called stem cells, and when needed, these stem cells can generate differentiated cells by division along developmental routes. The stem cell described in the present invention can be an embryonic stem cell, an adult stem cell (e.g., an umbilical cord blood stem cell, a bone marrow stem cell, a hematopoietic stem cell, a mesenchymal stem cell, etc.), and a pluripotent stem cell (e.g., an induced pluripotent stem cell iPSC, etc.).

In some embodiments, the expression of the endogenous HLA class I genes and/or HLA class II genes of the engineered cell of the present invention is not modified. That is, the expression level of any one of the endogenous HLA class I gene and/or HLA class II gene is not altered by any artificial intervention method (gene editing or non-gene editing).

In some embodiments, the expression of at least one of the endogenous HLA class I gene of the engineered cell of the present invention is suppressed or silenced. In some embodiments, the expression of at least one of the endogenous HLA class II gene of the engineered cell of the present invention is suppressed or silenced. In some embodiments, the expression of at least one of the endogenous TCR/CD3 gene of the engineered cell of the present invention is suppressed or silenced. In some embodiments, the expression of at least one of the endogenous TCR/CD3 gene and at least one of the endogenous HLA class I gene of the engineered cell of the present invention is suppressed or silenced. In some embodiments, the expression of at least one of the endogenous HLA class I gene and HLA class II gene of the engineered cell of the present invention is suppressed or silenced. In some embodiments, the expression of at least one of the endogenous TCR/CD3 gene, at least one of the endogenous HLA class I gene and at least one of the endogenous HLA class II gene of the engineered cell of the present invention is suppressed or silenced. Preferably, the HLA class I gene is selected from HLA-A, HLA-B, HLA-C and B2M. Preferably, the HLA class II gene is selected from HLA-DPA, HLA-DQ, HLA-DRA, TAP1, TAP2, LMP2, LMP7, RFX5, RFXAP, RFXANK and CIITA, preferably selected from RFX5, RFXAP, RFXANK and CIITA. Preferably, the TCR/CD3 gene is selected from TRAC, TRBC, CD3γ, CD3δ, CD3ε and CD3ζ.

In some embodiments, the expression of one or more endogenous genes of the engineered cell of the present invention is suppressed or silenced, the endogenous genes selected from the group consisting of: CD52, GR, dCK and immune checkpoint genes, e.g., PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3.

Methods for suppressing gene expression or silencing gene are well known to those skilled in the art, including but not limited to, for example, mediating DNA or RNA fragmentation by meganucleases, zinc finger nucleases, TALEN, CRISPR/Cas system, base editors, or inactivating genes through technologies, e.g., antisense oligonucleotides, RNAi, shRNA, transposons, mutaions, etc.

In some embodiments, the engineered cell of the present invention is an allogeneic cell. As used herein, the term "allogeneic" refers to any material derived from a different animal or a different patient of the same species as the individual into whom the material is introduced. Two or more individuals are considered allogeneic to each other when the genes at one or more loci differ. In some cases, allogeneic material from individuals of the same species may be genetically different enough for antigenic interactions to occur.

### Functional exogenous receptors

In some embodiments, the engineered cell expressing the immunosuppressive molecule of the present invention can further express a functional exogenous receptor. The functional exogenous receptor of the present invention comprises a chimeric antigen receptor (CAR), a T cell receptor (TCR), a T cell receptor fusion protein (TFP), a T cell antigen coupler (TAC) or an immune mobilization monoclonal T cell receptor (ImmTAC), etc., preferably a chimeric antigen receptor or a T cell receptor, more preferably a chimeric antigen receptor.

The term "T cell receptor" or "TCR" refers to a membrane protein complex that responds to antigen presentation and participates in T cell activation. The stimulation of TCR is triggered by the major histocompatibility complex molecule (MHC) on the antigen presenting cell, which presents antigen peptides to T cells and bind to the TCR complexs to induce a series of intracellular signaling. TCR is composed of six peptide chains that form heterodimers, which are generally divided into αβ type and γδ type. Each peptide chain includes a constant region and a variable region, wherein the variable region is responsible for binding to specific antigen and MHC molecules. The variable region of TCR may comprise a ligand binding domain or be operably linked to a ligand binding domain, wherein the definition of the ligand binding domain is as described below.

The term "chimeric antigen receptor" or "CAR" refers to an artificially constructed hybrid polypeptide that generally includes an antigen (e.g., a tumor antigen) binding domain (e.g., an antibody or antigen ligand), a transmembrane domain, an optional co-stimulatory domain, and a primary signaling domain, and each domain is linked by a linker. CARs are able to reposition the specificity and reactivity of T cells and other immune cells to a selected target in a non-MHC-restricted manner. In some embodiments, the functional exogenous receptor of the present invention is a chimeric antigen receptor, which comprises a tumor antigen binding domain, a transmembrane domain, one or more co-stimulatory domains, and a primary signaling domain. In some embodiments, the chimeric antigen receptor further comprises one or more of the following structures: a signal peptide, a hinge region, a suicide gene, a switch structure, etc.

The term "T cell antigen coupler" or "TAC" includes three functional domains: (1) a tumor targeting domain, including a single chain antibody, a designed ankyrin repeat protein (DARPin), or other targeting groups; (2) an extracellular domain, a CD3-binding single-chain antibody, so that TAC receptors and TCR receptors are close to each other; and (3) a transmembrane domain and an intracellular domain of a CD4 co-receptor, wherein the intracellular domain is linked to protein kinase LCK, to catalyze the phosphorylation of the immunoreceptor tyrosine activation motif (ITAM) of the TCR complex as an initial step in T cell activation.

The term "T cell receptor fusion protein" or "TFP" refers to a recombinant polypeptide derived from components of TCR, usually composed of a TCR subunit and an antigen binding region linked thereto, and expressed on the cell surface. Wherein, the TCR subunit includes at least part of the TCR extracellular domain, the transmembrane domain, and the TCR intracellular signal domain.

The term "immune mobilizing monoclonal T cell receptor" or "ImmTAC" is composed of an engineered T cell receptor (TCR) and an anti-CD3 scFv, wherein: the engineered TCR can specifically recognize and bind to HLA-peptide complex on the surface of tumor cells with significantly improved affinity, and promotes T cell-mediated effector functions through the interaction of the scFv antibody fragment with CD3.

In some embodiments, the functional exogenous receptor comprises an extracellular domain that specifically recognizes an antigen (e.g., a tumor antigen). In some embodiments, the extracellular domain comprises an antibody that specifically binds an antigen or a ligand of the antigen. In some embodiments, the antigen is selected from the group consisting of: ALK, ADRB3, AKAP-4, APRIL, ASGPR1, BCMA, B7H3, B7H4, B7H6, bcr-abl, BORIS, BST2, BAFF-R, BTLA, CD2, CD3, CD4, CD5, CD7, CD8, CD19, CD20, CD22, CD24, CD25, CD28, CD30, CD33, CD38, CD40, CD44, CD44v6, CD44v7/8, CD47, CD52, CD56, CD57, CD58, CD70, CD72, CD79a, CD79b, CD80, CD81, CD86, CD97, CD123, CD133, CD137, CD 138, CD151, CD171, CD179a, CD300LF, CDH16, CSPG4, CS1, Claudin 6, Claudin18.1, Claudin 18.2, CEA, CEACAM6, CLL1, c-Met, CAIX, CXORF61, CA125, CYP1B1, CS1, ELF2M, EGFR, EPCAM, EGFRvIII, EphA2, ERG/TMPRSS2ETS fusion gene, ETV6-AML, EMR2, EGP2, EGP40, FAP, FAR, FBP, FLT3, FOSL1, FCRL5, FCAR, Flt3, Flt4, Frizzled, GD2, GD3, gp100, gp130, GM3, GPC2, GPC3, GPRC5D, GPR20, GloboH, GHRHR, GHR, GITR, Her2, HER3, HER-4, HMWMAA, HAVCR1, HPV E6,E7, HVEM, HIV-1Gag, HLA-A1, HLA-A2, IL6R, IL-11Ra, IL-13Ra, IGF-I receptor, LTPR, LIFRP, LRP5, IGLL1, IGF1R, KIT, Kappa Light Chain, KDR, LewisY, LMP2, LY6K, LAGE-1a, legumain, LCK, LAIR1, LILRA2, LY75, MSLN, MUC1, MUC16, MAGE-A1, MAGE3, MAD-CT-1, MelanA/MART1, ML-IAP, MYCN, mut hsp70-2, NCAM, NY-BR-1, NY-ESO-1, NA17, Notch-1-4, nAchR, NKG2D, NKG2D ligand, OY-TES1, OR51E2, OX40, PRSS21, PSCA, PD1, PD-L1, PD-L2, PSMA, Prostase, PAP, PDGFR-β, PCTA-1/galectin 8, p53, p53 mutant, prostein, PLAC1, PANX3, PAX3, PAX5, PTCH1, RANK, RAGE-1, ROR1, Ras mutant, RhoC, RU1, RU2, Robol, SSEA-4, SSX2, SART3, Sp17, TSHR, Tn Ag, TGS5, TEM1/CD248, TEM7R, TARP, TCRα, TCRβ, TGFBR1, TGFBR2, TNFRSF4, TWEAK-R, TLR7, TLR9, TAG72, TROP-2, Tie 2, TRP-2, TNFR1, TNFR2, TEM1, UPK2VEGFR, WT1, XAGE1, 5T4, 8H9, αvβ6 integrin, CA9, folate receptor α, ephrin B2, tyrosinase, fucosyl GM1, o-acetyl-GD2, folate receptor β, polysialic acid, sperm protein 17, survivin and telomerase, sarcoma translocation breakpoint, human telomerase reverse transcriptase/hTERT, androgen receptor, intestinal carboxylesterase, cyclin B1, fibronectin, tenascin, carcinoembryonic variant of tumor necrosis region, and any combination thereof. Preferably, the antigen is selected from CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin 18.2, ROR1, EGFRvIII, CS1, BCMA and GPRC5D, more preferably selected from CD19, Claudin 18.2, MSLN, GPRC5D, ROR1, CD7 and BCMA.

In some embodiments, the functional exogenous receptor comprises an extracellular domain that specifically recognizes CD19, for example, a CD19-targeting antibody. CD19-targeting antibodies known in the art can all be used in the present invention. In some embodiments, the CD19-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 103; and the CDR1-L, CDR2-L and CDR3-L comprised in the light chain variable region are the same as the CDR1-L, CDR2-L and CDR3-L comprised in SEQ ID NO: 104. In some embodiments, CDR1-H comprised in the heavy chain variable region is as set forth in SEQ ID NO: 97, CDR2-H is as set forth in SEQ ID NO: 98, and CDR3-H is as set forth in SEQ ID NO: 99; CDR1-L comprised in the light chain variable region is as set forth in SEQ ID NO: 100, CDR2-L is as set forth in SEQ ID NO: 101, and CDR3-L is as set forth in SEQ ID NO: 102.

In some embodiments, the CD19-targeting antibody comprises a light chain variable region and a heavy chain variable region, wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 103; wherein the light chain variable region has at least 90%, at least 91%, at least 92%, at least 93%, at least 94%, at least 95%, at least 96%, at least 97%, at least 98%, at least 99%, or 100% identity to SEQ ID NO: 104. Preferably, the CD19-targeting antibody of the present invention comprises a heavy chain variable region as set forth in SEQ ID NO: 103 and a light chain variable region as set forth in SEQ ID NO:104.

In some embodiments, the CD19-targeting antibody has at least 90%, at least 91%, at least 92%, at least 93%, at least 94%, at least 95%, at least 96%, at least 97%, at least 98%, at least 99%, or 100% identity to SEQ ID NO: 105. Preferably, the CD19-targeting antibody is as set forth in SEQ ID NO: 105.

In some embodiments, the functional exogenous receptor comprises an extracellular domain that specifically recognizes Claudin 18.2, for example, a Claudin 18.2-targeting antibody. Claudin 18.2-targeting antibodies known in the art can all be used in the present invention. In some embodiments, the Claudin 18.2-targeting antibody comprises a VHH, wherein the CDR1, CDR2 and CDR3 comprised in the VHH are the same as CDR1, CDR2 and CDR3 as comprised in SEQ ID NO: 109. In some embodiments, CDR1 comprised in the VHH is as set forth in SEQ ID NO: 106, CDR2 is as set forth in SEQ ID NO: 107, and CDR3 is as set forth in SEQ ID NO: 108.

In some embodiments, the Claudin 18.2-targeting antibody comprises an amino acid sequence having at least 90%, at least 91%, at least 92%, at least 93%, at least 94%, at least 95%, at least 96%, at least 97%, at least 98%, at least 99%, or 100% identity to SEQ ID NO: 109. Preferably, the Claudin 18.2-targeting antibody of the present invention comprises the amino acid sequence as set forth in SEQ ID NO: 109.

In some embodiments, the functional exogenous receptor of the present invention is a chimeric antigen receptor, which comprises an antigen binding domain, a transmembrane domain, one or more co-stimulatory domains, and a primary signaling domain. In some embodiments, the functional exogenous receptor of the present invention is a chimeric antigen receptor, the chimeric antigen receptor targeting CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin 18.2, ROR1, EGFRvIII, CS1, BCMA, GPRC5D, or any combination thereof, more preferably targeting CD19, Claudin 18.2, MSLN, ROR1, GPRC5D, CD7, BCMA, or any combination thereof. In some embodiments, the chimeric antigen receptor further comprises a signal peptide, a hinge region, or both. For the definitions of the transmembrane domain, co-stimulatory domain, primary signaling domain, and the optional hinge region, signal peptide and other structures that can be used in the CAR of the present invention, see the above "immunosuppressive molecules" section.

In some embodiments, the CAR of the present invention may further comprise a switch structure to regulate the expression time of the CAR. For example, the switch structure may be in the form of a dimerization domain, which undergoes a conformational change by binding to its corresponding ligand, exposing the extracellular binding domain, allowing it to bind to the targeted antigen, thereby activating the signal transduction pathway. Alternatively, switch domains may also be used to link the binding domain and the signaling domain, respectively, and only when the switch domains are coupled with each other (e.g., in the presence of an inducing compound), the binding domain and the signaling domain can be linked together through the dimerization, thereby activating the signaling pathway. The switch structure may further be in the form of a masking peptide. The masking peptide can mask the extracellular binding region and prevent it from binding to the targeted antigen. When the masking peptide is cleaved by, for example, protease, the extracellular binding region can be exposed, making it a "normal" CAR structure. Various switch structures known to those skilled in the art can all be used in the present invention.

In some embodiments, the CAR of the present invention may further comprise a suicide gene, i.e., to make it express a cell death signal that can be induced by an exogenous substance, so as to eliminate CAR cells when needed (e.g., when severe toxic side effects occur). For example, the suicide gene may be in the form of inserted epitopes, for example, CD20 epitopes, RQR8, etc., and when necessary, CAR cells can be eliminated by adding antibodies or reagents targeting these epitopes. The suicide gene may also be herpes simplex virus thymidine kinase (HSV-TK), which could cause cell death induced by ganciclovir treatment. The suicide gene may further be iCaspase-9, and the dimerization of iCaspase-9 can be induced by a chemically inducing medicament, e.g., AP1903, AP20187, etc., thereby activating the downstream Caspase3 molecule and leading to cell apoptosis. Various suicide genes known to those skilled in the art can all be used in the present invention.

### IV. Method for suppressing immune rejection of exogenous cells by immune cells of a subject

In one aspect, the present invention further provides a method for reducing immune rejection of exogenous cells by cells of a subject, including the following step: expressing the immunosuppressive molecule, the nucleic acid molecule encoding the immunosuppressive molecule or the vector comprising the nucleic acid molecule of the present invention in the exogenous cells.

In another aspect, the present invention further provides a method for reducing immune rejection of exogenous cells by cells of a subject, including the following step: administering the immunosuppressive molecule or the engineered cell expressing the immunosuppressive molecule of the present invention to the subject. In some embodiments, the immunosuppressive molecule or the engineered cell of the present invention is administered simultaneously or sequentially with the exogenous cells. For example, the immunosuppressive molecule or the engineered cell of the present invention can be administered after the exogenous cells.

The term "subject" is a mammal. Mammals can be humans, non-human primates, mice, rats, dogs, cats, pigs, horses, or cows, but are not limited to these examples. Mammals other than humans can be advantageously used as subjects representing animal models of cancer. Preferably, the subject is a human.

### V. Pharmaceutical composition

The present invention further provides a pharmaceutical composition, which comprises the immunosuppressive molecule, the engineered cell, the nucleic acid molecule or vector of the present invention as an active agent, and one or more pharmaceutically acceptable excipients. Therefore, the present invention further encompasses use of the immunosuppressive molecule, the nucleic acid molecule, the vector or the engineered cell in the preparation of a pharmaceutical composition or a medicament.

The term "pharmaceutically acceptable excipient" refers to a vector and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, co-solvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present invention. Exemplary excipients for use in the pharmaceutical composition of the present invention include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present invention is suitable for multiple routes of administration. Generally, the administration is parenterally accomplished. Parenteral delivery methods include topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present invention may also be prepared in various forms, e.g., solid, liquid, gaseous or lyophilized forms, and particularly may be in a form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present invention for producing a medicament may include, for example, conventional mixing, dissolving, granulating, sugar-coating, grinding, emulsifying, encapsulating, embedding or lyophilizing process. The pharmaceutical composition comprising, for example, the immune cell as described herein is generally provided in a form of solution, and preferably comprises a pharmaceutically acceptable buffer agent.

The pharmaceutical composition according to the present invention further may be administered in combination with one or more other agents (biological agents, e.g., antibody reagents, and/or small molecules) or treatment methods (e.g., surgery, chemotherapy or radiotherapy) suitable for the treatment and/or prevention of diseases to be treated. Preferred examples of an agent suitable for the combination include known anti-cancer medicaments, e.g., cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, e.g., ricin, diphtheria toxin, pseudomonas exotoxin A, DNase and RNase; radionuclides, e.g., iodine 131, rhenium 186, indium 111, iridium 90, bismuth 210, bismuth 213, actinides 225 and astatine 213; prodrugs, e.g., antibody-directed enzyme prodrugs; immunostimulatory agents, e.g., platelet factor 4, melanoma growth stimulating protein, etc.; antibodies or fragments thereof, e.g., anti-CD3 antibodies or fragments thereof, complement activators, heterologous protein domains, homologous protein domains, viral/bacterial protein domains and viral/bacterial peptides. In addition, the pharmaceutical composition of the present invention also can be used in combination with one or more other treatment methods, for example, chemotherapy and radiotherapy.

### VI. Use and treatment methods

The present invention further provides use of the pharmaceutical composition or the engineered cell described above in the preparation of a medicament for diseases such as cancer, infection or autoimmunity, and a method for treating cancer, infection or autoimmune diseases.

In some embodiments, the cancer is cancer associated with the expression of a target binding to a functional exogenous receptor. For example, the cancer includes, but is not limited to, brain glioma, blastoma, sarcoma, leukemia, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of the digestive system, endometrial cancer, esophageal cancer, eye cancer, head and neck cancer, gastric cancer (including gastrointestinal cancer), glioblastoma (GBM), liver cancer, hepatoma, intraepithelial neoplasia, kidney cancer, laryngeal cancer, liver tumor, lung cancer (e.g., small cell lung cancer, non-small cell lung cancer, adenocarcinoma of the lung, and squamous cell lung cancer), lymphoma (including Hodgkin's lymphoma and non-Hodgkin's lymphoma), melanoma, myeloma, neuroblastoma, oral cancer (e.g., lip, tongue, mouth, and pharynx), ovarian cancer, pancreatic cancer, prostate cancer, retinoblastoma, rhabdomyosarcoma, colorectal cancer, cancer of the respiratory system, salivary gland cancer, skin cancer, squamous cell carcinoma, gastric cancer, testicular cancer, thyroid cancer, uterine or endometrial cancer, malignancy of the urinary system, vulvar cancer, and other cancers and sarcomas, and B-cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, large mass disease NHL), mantle cell lymphoma, AIDS-related lymphoma, and Waldenstrom macroglobulinemia, chronic lymphocytic leukemia (CLL), acute lymphoblastic leukemia (ALL), B-cell acute lymphoblastic leukemia (B-ALL), T-cell acute lymphoblastic leukemia (T-ALL), B-cell prolymphocytic leukemia, blastic plasmacytoid dendritic cell neoplasm, Burkitt's lymphoma, diffuse large B-cell lymphoma, follicular lymphoma, chronic myeloid leukemia (CML), malignant lymphoproliferative disease, MALT lymphoma, hairy cell leukemia, marginal zone lymphoma, multiple myeloma, myelodysplasia, plasmablastic lymphoma, preleukemia, plasmacytoid dendritic cell neoplasm, and post-transplant lymphoproliferative disorder (PTLD); and other diseases associated with target expression. Preferably, the disease that can be treated with the engineered cell or the pharmaceutical composition of the present invention is selected from the group consisting of: leukemia, lymphoma, multiple myeloma, brain glioma, pancreatic cancer, gastric cancer, etc.

In some embodiments, the infection includes, but is not limited to, infections caused by viruses, bacteria, fungi, and parasites.

In some embodiments, the autoimmune disease includes, but is not limited to type I diabetes, celiac disease, Graves' disease, inflammatory bowel disease, multiple sclerosis, psoriasis, rheumatoid arthritis, Addison's disease, Sjögren's syndrome, Hashimoto's thyroiditis, myasthenia gravis, vasculitis, pernicious anemia and systemic lupus erythematosus.

The present invention will be described in detail below with reference to the accompanying drawings and examples. It should be noted that those skilled in the art should understand that the drawings of the present invention and the examples thereof are only for the purpose of illustration, and shall not constitute any limitation to the present invention. In the case of no contradiction, the embodiments in the present invention and the features in the embodiments can be combined with each other.

### BRIEF DESCRIPTION OF DRAWINGS

Figure 1: CAR expression levels in CAR19-AT3 T, CAR19-AL3 T, CAR19-ATT T, and CAR19-A T cells.
Figure 2: anti-LAG3 scFv and anti-TIGIT scFv expression levels in CAR19-AL3 T and CAR19-ATT T cells.
Figure 3: Cytokine release levels in CAR19-AT3 T, CAR19-AL3 T, CAR19-ATT T, and CAR19-A T cells after co-incubated with target cell Nalm6.
Figure 4: Suppressive effects of CAR19-AT3 T, CAR19-AL3 T, CAR19-ATT T, and CAR19-A T cells on allogeneic PBMC proliferation.
Figure 5: CAR expression levels in CAR19-AC4 T and CAR19-A T cells.
Figure 6: anti-NKG2A scFv and anti-CTLA4 scFv expression levels in CAR19-AC4 T and CAR19-A T cells.
Figure 7: Cytokine release levels in CAR19-AC4 T and CAR19-A T cells after co-incubation with target cell Nalm6.
Figure 8: Suppressive effects of CAR19-AC4 T and CAR19-A T cells on allogeneic PBMC proliferation.
Figure 9: CAR expression levels in CAR19-C4T3 T, CAR19-C4L3 T, CAR19-C4TT T and CAR19-C4 T cells.
Figure 10: Anti-CTLA4 scFv, anti-LAG3 scFv or anti-TIGIT scFv expression levels in CAR19-C4T3 T, CAR19-C4L3 T, CAR19-C4TT T and CAR19-C4 T cells.
Figure 11: Cytokine release levels in CAR19-C4T3 T, CAR19-C4L3 T, CAR19-C4TT T and CAR19-C4 T cells after co-incubation with target cell Nalm6.
Figure 12: Suppressive effects of CAR19-C4T3 T, CAR19-C4L3 T, CAR19-C4TT T and CAR19-C4 T cells on allogeneic PBMC proliferation.
Figure 13: CAR expression levels in CAR19-PT3 T, CAR19-PL3 T, CAR19-PC4 T and CAR19-P T cells.
Figure 14: Anti-LAG3 scFv, anti-TIGIT scFv or anti-CTLA4 scFv expression levels in CAR19-PT3 T, CAR19-PL3 T, CAR19-PC4 T and CAR19-P T cells.
Figure 15: Cytokine release levels in CAR19-PT3 T, CAR19-PL3 T, CAR19-PC4 T and CAR19-P T cells after co-incubation with target cell Nalm6.
Figure 16: Suppressive effects of CAR19-PT3 T, CAR19-PL3 T, CAR19-PC4 T and CAR19-P T cells on allogeneic PBMC proliferation.
Figure 17: CAR expression levels in CAR18.2-PA T, CAR18.2-PE T and CAR18.2-P T cells.
Figure 18: Anti-NKG2A scFv or anti-PD1 scFv expression levels in CAR18.2-PA T, CAR18.2-PE T and CAR18.2-P T cells.
Figure 19: Killing effects of CAR18.2-PA T, CAR18.2-PE T and CAR18.2-P T cells on target cell Claudin 18.2/NUGC4.
Figure 20: In vivo tumor suppression of CAR18.2-PA T, CAR18.2-PE T and CAR18.2-P T cells.
Figure 21: CAR expression levels in CAR18.2 T, CAR18.2-PC T, CAR18.2-CA T, CAR18.2PTT T, CAR18.2-PL3 T and CAR18.2-PT3 T cells.
Figure 22: PDL1, anti-TIGIT scFv or anti-LAG3 scFv expression levels in CAR18.2-PTT T, CAR18.2-PL3 T and CAR18.2-PT3 T cells.
Figure 23: Killing effects of CAR18.2 T, CAR18.2-PC T, CAR18.2-CA T, CAR18.2PTT T, CAR18.2-PL3 T and CAR18.2-PT3 T cells on target cell Claudin 18.2/NUGC4;
Figure 24: PBMC expansion of allogeneic PBMC after 10 days of co-culture with CAR18.2 T, CAR18.2-PC T, CAR18.2-CA T, CAR18.2PTT T, CAR18.2-PL3 T or CAR18.2-PT3.

### Examples

### Example 1: Construction of anti-CD19 CAR-T cells expressing the immunosuppressive molecule comprising an NKG2A binding domain and verification of the function thereof

Sequences encoding the following were synthesized and sequentially cloned into the pGEM-T Easy vector (Promega): anti-CD19 scFv (SEQ ID NO: 105), CD8α hinge region (SEQ ID NO: 86), CD8α transmembrane region (SEQ ID NO: 83), 4-1BB co-stimulatory domain (SEQ ID NO: 90), CD3ζ intracellular region (SEQ ID NO: 92) to obtain a CAR19 plasmid, and the correct insertion of the target sequence in the plasmid was confirmed by sequencing.

The immunosuppressive molecule linked by T2A (SEQ ID NO: 110) was further expressed in the above CAR19 plasmid, and its structure was as follows: B2M signal peptide (SEQ ID NO: 94), immunosuppressive protein binding domain (Table 1), IgG4 hinge region (SEQ ID NO: 88), CD28 transmembrane region (SEQ ID NO: 82), CD28 co-stimulatory domain (SEQ ID NO: 89) to obtain plasmids CAR19-A, CAR19-AT3, CAR19-AL3, CAR19-ATT, and CAR19-AC4, and the correct insertion of the target sequence in the plasmid was confirmed by sequencing.

**Table 1 Plasmids expressing the immunosuppressive molecule comprising an NKG2A binding domain**

| CAR target | Immunosuppressive protein binding domain | Plasmid |
|---|---|---|
| CD19 | Anti-NKG2A scFv (SEQ ID NO:9) | CAR19-A |
| CD19 | Anti-NKG2A scFv (SEQ ID NO:9), linker (SEQ ID NO:111), anti-TIM3 scFv (SEQ ID NO:21) | CAR19-AT3 |
| CD19 | Anti-NKG2A scFv (SEQ ID NO:9), linker (SEQ ID NO:111), anti-LAG3 scFv (SEQ ID NO:34) | CAR19-AL3 |
| CD19 | Anti-NKG2A scFv (SEQ ID NO:9), linker (SEQ ID NO:111), anti-TIGIT scFv (SEQ ID NO:47) | CAR19-ATT |
| CD19 | Anti-NKG2A scFv (SEQ ID NO:9), linker (SEQ ID NO:111), anti-CTLA4 scFv (SEQ ID NO:64) | CAR19-AC4 |

### 1.1 Construction of CAR19-AT3 T, CAR19-AL3 T, and CAR19-ATT T cells and verification of the functions thereof

### 1.1.1 Construction of CAR19-AT3 T, CAR19-AL3 T, and CAR19-ATT T cells

Three ml Opti-MEM (Gibco) was added to a sterile tube to dilute the CAR19-A, CAR19-AT3, CAR19-AL3, and CAR19-ATT plasmids, respectively, and then packaging vector psPAX2 (Addgene) and envelope vector pMD2.G (Addgene) were added according to the ratio of plasmid : viral packaging vector : viral envelope vector = 4:2:1. Then, 120 µl X-treme GENE HP DNA transfection reagent (Roche) was added, mixed immediately, and incubated at room temperature for 15 min, and then the plasmid/vector/transfection reagent mixture was added dropwise to the culture flask of 293T cells. Viruses were collected at 24 hours and 48 hours, pooled, and ultracentrifuged (25000 g, 4°C, 2.5 hours) to obtain concentrated lentiviruses.

T cells were activated with DynaBeads CD3/CD28 CTSTM (Gibco), and were further cultured for 1 day under 37°C and 5% CO₂. On the second day, the concentrated lentiviruses were added, and after 3 days of continuous culture, T cells expressing CD19 and the immunosuppressive molecule (CAR19-A T, CAR19-AT3 T, CAR19-AL3 T, and CAR19-ATT T cells) were obtained.

### 1.1.2 Detection of CAR expression levels and immunosuppressive protein binding domain expression levels in various CAR-T cells

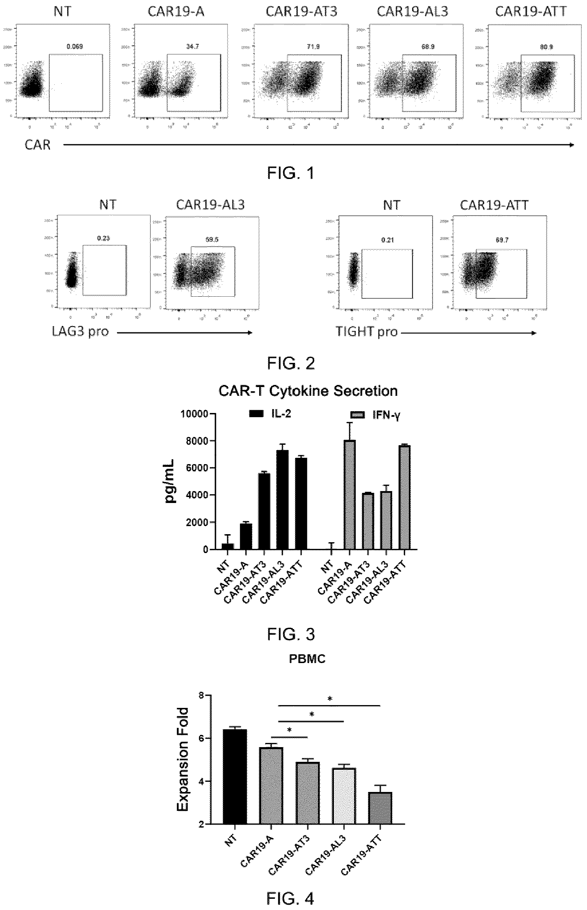

Using a flow cytometry, the expression of anti-CD19 scFv in CAR19-A T, CAR19-AT3 T, CAR19-AL3 T, and CAR19-ATT T cells was detected with FITC goat anti-human Fab (Thermo Fisher). It can be seen that all the CARs in the CAR-T cells prepared in this example can be effectively expressed (Figure 1).

Using a flow cytometry, the expression of anti-LAG3 scFv in CAR19-AL3 T cells was detected with Human LAG-3 / CD223 Protein, His Tag (ACRO); and the expression of anti-TIGIT scFv in CAR19-ATT T cells was detected with Human TIGIT Protein, Fc TagTIGIT (ACRO). It can be seen that both anti-LAG3 scFv in CAR19-AL3 T cells and anti-TIGIT scFv in CAR19-ATT T cells can be effectively expressed (Figure 2).

### 1.1.3 Detection of cytokine release levels in various CAR-T cells

Target cells Nalm6 were plated in 96-well plates at a concentration of 1×10⁵ cells/well, CAR19-A T cells, CAR19-AT3 T, CAR19-AL3 T, CAR19-ATT T, and NT cells were added at a ratio of 1:1, respectively, and the cell co-culture supernatant was collected after 18-24 hours of co-culture.

Human IL-2 DuoSet ELISA Kit (R&D systems) and Human IFN-gamma DuoSet ELISA Kit (R&D systems) were used to detect the contents of IL2 and IFN-γ in the co-culture supernatant, respectively, and the results are shown in Figure 3.

It can be seen that compared with NT cells, after co-culture with target cells, the release levels of cytokines IL2 and IFN-γ of CAR19-A T cells, CAR19-AT3 T, CAR19-AL3 T, and CAR19-ATT T cells are significantly increased, indicating that this cytokine release is specific. More importantly, compared with CAR19-A T cells expressing the immunosuppressive molecule targeting only NKG2A, CAR19-AT3 T, CAR19-AL3 T, and CAR19-ATT T cells expressing the immunosuppressive molecule targeting a combination of NKG2A and other immunosuppressive proteins have a stronger promoting effect on IL2 release by CAR T cells.

### 1.1.4 Detection of the suppressive effects of various CAR-T cells on HvGD in vitro

The suppressive effects of various CAR-T cells prepared in 1.1.1 on HvGD in vitro were assessed by one-way mixed lymphocyte reaction (MLR) assay. Specifically, NT cells, CAR19-A T cells, CAR19- AT3 T cells, CAR19-AL3 T cells, and CAR19-ATT T cells were co-incubated with allogeneic PBMCs under 37°C and 5% CO₂ for 7 days, and the proliferation of PBMCs was then detected by flow cytometry, and the results are shown in Figure 4.

It can be seen that compared with NT cells, all the CAR T cells that additionally express the immunosuppressive molecule of the present invention can significantly suppress the proliferation of allogeneic PBMCs. In particular, it is worth noting that compared with CAR T cells with the immunosuppressive molecule targeting only NKG2A (CAR19-A T), CAR T cells expressing the immunosuppressive molecule targeting both NKG2A and TIM3, NKG2A and LAG3, or NKG2A and TIGIT (CAR19-AT3 T, CAR19-AL3 T, CAR19-ATT T) further significantly suppress the proliferation of allogeneic PBMCs.

### 1.2. Construction of CAR19-AC4 T cells and verification of the function thereof

### 1.2.1 Construction of CAR19-AC4 T cells

According to the method in 1.1.1, plasmids CAR19-A and CAR19-AC4 were transferred into T cells derived from another donor, respectively, to obtain T cells expressing CAR19 and the immunosuppressive molecule (CAR19-A T, CAR19-AC4 T cells).

### 1.2.2 Detection of CAR expression levels and immunosuppressive protein binding domain expression levels in CAR19-AC4 T

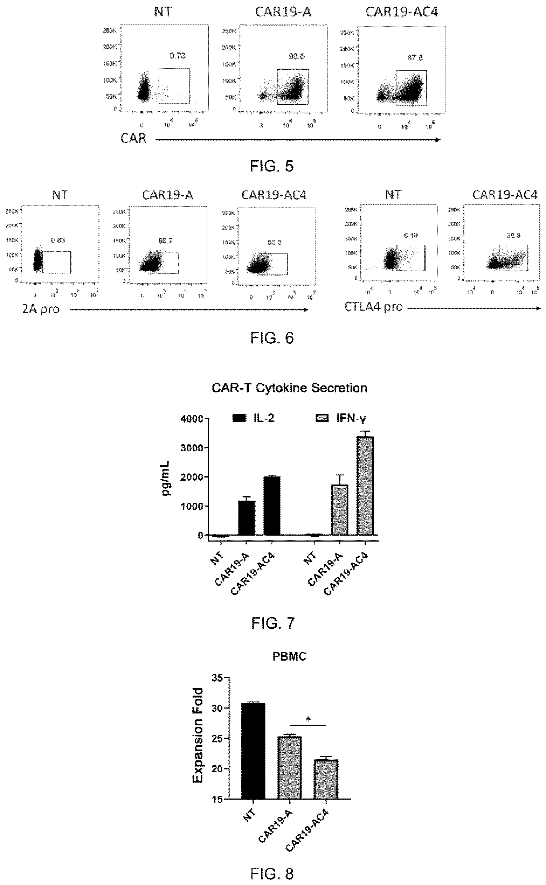

Using a flow cytometry, the expression of anti-CD19 scFv in CAR19-A T and CAR19-AC4 T cells was detected with FITC goat anti-human Fab (Thermo Fisher). It can be seen that the CARs in both CAR19-A T and CAR19-AC4 T cells can be effectively expressed (Figure 5).

Using a flow cytometry, the expression of anti-NKG2A scFv in CAR19-A T and CAR19-AC4 T cells was detected with Human NKG2A&CD94 Protein, Fc Tag (ACRO); and the expression of anti-CTLA-4 scFv in CAR19-AC4 T cells was detected with Human CTLA-4 / CD152 Protein, Fc Tag (ACRO). It can be seen that both anti-NKG2A scFv and anti-CTLA4 scFv in CAR19-AC4 T cells can be effectively expressed (Figure 6).

### 1.2.3 Detection of cytokine release levels in CAR19-AC4 T cells

According to the detection method in 1.1.3, cytokine release levels of NT, CAR19-AT and CAR19-AC4 cells were detected and the results are shown in Figure 7.

It can be seen that compared with NT cells, after co-culture with target cells, the release levels of both cytokines IL2 and IFN-γ in CAR19-A T and CAR19-AC4 T cells are significantly increased, indicating that this cytokine release is specific; compared with CAR19-A T cells, the release levels of both cytokines IL2 and IFN-γ in CAR19-AC4 cells are significantly increased, indicating that compared with the immunosuppressive molecule targeting only NKG2A, the immunosuppressive molecule targeting NKG2A and CTLA4 have a stronger promoting effect on the release of IL2 and IFN-γ in CAR T cells.

### 1.2.4 Detection of the suppressive effect of CAR19-AC4 T on HvGD in vitro

According to the detection method in 1.1.4, the suppressive effects of CAR19-A T and CAR19-AC4 T cells on HvGD in vitro were assessed by one-way mixed lymphocyte reaction (MLR) assay.

It can be seen that compared with NT cells, CAR19-A T and CAR19-AC4 T can significantly suppress the proliferation of allogeneic PBMCs; compared with CAR19-A T cells, CAR T cells expressing the immunosuppressive molecule targeting NKG2A and CTLA4 (CAR19-AC4 T) further significantly suppress the proliferation of allogeneic PBMCs.

The above results indicate that compared with the immunosuppressive molecule targeting only NKG2A, the immunosuppressive molecules targeting NKG2A and other immunosuppressive proteins (such as TIM3, LAG3, TIGIT or CTLA4) can significantly suppress the in vitro proliferation of allogeneic PBMCs, thereby significantly reducing HvGD, and have a stronger promoting effect on the release of cytokines (particularly IL2) after co-culture of CAR T cells with target cells.

### Example 2: Construction of anti-CD19 CAR-T cells expressing the immunosuppressive molecule that comprises a CTLA4 binding domain and verification of the function thereof

### 2.1 Construction of anti-CD19 CAR-T cells expressing the immunosuppressive molecule comprising a CTLA4 binding domain

The immunosuppressive molecule linked by T2A (SEQ ID NO: 110) was further expressed in the CAR19 plasmid prepared in Example 1, and its structure was as follows: B2M signal peptide (SEQ ID NO: 94), immunosuppressive protein binding domain (Table 2), IgG4 hinge region (SEQ ID NO: 88), CD28 transmembrane region (SEQ ID NO: 82), CD28 co-stimulatory domain (SEQ ID NO: 89) to obtain plasmids CAR19-C4, CAR19-C4T3, CAR19-C4L3, and CAR19-C4TT, and the correct insertion of the target sequence in the plasmid was confirmed by sequencing.

**Table 2 Plasmids expressing the immunosuppressive molecule comprising an CTLA4 binding domain**

| CAR target | Immunosuppressive protein binding domain | Plasmid |
|---|---|---|
| CD19 | Anti-CTLA4 scFv (SEQ ID NO:64) | CAR19-C4 |
| CD19 | Anti-CTLA4 scFv (SEQ ID NO:64), linker (SEQ ID NO:111), anti-TIM3 scFv (SEQ ID NO:21) | CAR19-C4T3 |
| CD19 | Anti-CTLA4 scFv (SEQ ID NO:64), linker (SEQ ID NO:111), anti-LAG3 scFv (SEQ ID NO:34) | CAR19-C4L3 |
| CD19 | Anti-CTLA4 scFv (SEQ ID NO:64), linker (SEQ ID NO:111), anti-TIGIT scFv (SEQ ID NO:47) | CAR19-C4TT |

According to the method in 1.1.1, plasmids CAR19-C4, CAR19-C4T3, CAR19-C4L3 and CAR19-C4TT were transferred into T cells respectively, to obtain CAR19-C4 T, CAR19-C4T3 T, CAR19-C4L3 T and CAR19-C4TT T cells.

### 2.2 Detection of CAR expression levels and immunosuppressive protein binding domain expression levels in various CAR-T cells

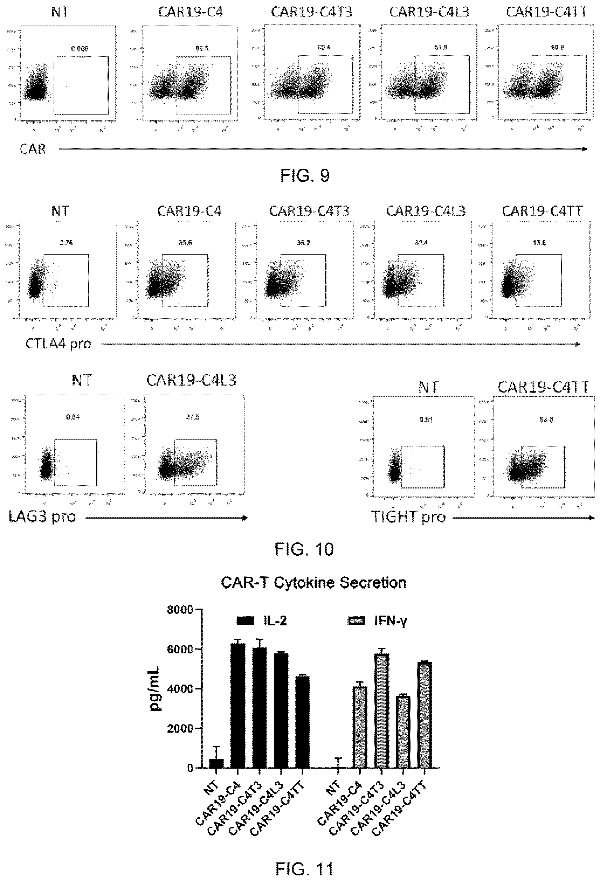

Using a flow cytometry, the expression of anti-CD19 scFv in CAR19-C4 T, CAR19-C4T3 T, CAR19-C4L3 T, and CAR19-C4TT T cells was detected with FITC goat anti-human Fab (Thermo Fisher). It can be seen that all the CARs in the CAR-T cells prepared in this example can be effectively expressed (Figure 9).

Using a flow cytometry, the expression of anti-CTLA4 scFv in CAR19-C4 T, CAR19-C4T3 T, CAR19-C4L3 T, and CAR19-C4TT T cells was detected with Human CTLA-4 / CD152 Protein, Fc Tag (ACRO); the expression of anti-LAG3 scFv in CAR19-C4L3 T was detected with Human LAG-3 / CD223 Protein, His Tag (ACRO); and the expression of anti-TIGIT scFv in CAR19-C4TT T was detected withs Human TIGIT Protein, Fc Tag (ACRO). It can be seen that anti-CTLA4 scFv in CAR19-C4 T, CAR19-C4T3 T, CAR19-C4L3 T, and CAR19-C4TT T cells, anti-LAG3 scFv in CAR19-C4L3 T, and anti-TIGIT scFv in CAR19-C4TT T cells can all be effectively expressed (Figure 10).

### 2.3 Detection of cytokine release levels in various CAR-T cells

According to the method in Example 1.1.3, the contents of IL2 and IFN-γ in the co-culture supernatant of CAR-T cells and target cells were detected and the results are shown in Figure 11.

It can be seen that compared with NT cells, after co-culture with target cells, the release levels of both cytokines IL2 and IFN-γ in CAR19-C4 T, CAR19-C4T3 T, CAR19-C4L3 T and CAR19-C4TT T cells are significantly increased, indicating that this cytokine release is specific; while compared with CAR19-C4 T cells, the release levels of CAR19-C4T3 T, CAR19-C4L3 T and CAR19-C4TT T cells are not much different, indicating that compared with the immunosuppressive molecule targeting only CTLA4, the immunosuppressive molecule targeting a combination of CTLA4 and other immunosuppressive proteins (such as TIM3, LAG3 or TIGIT) has no obvious adverse effect on the cytokine release from CAR T cells.

### 2.4 Suppressive effect on HvGD in vitro

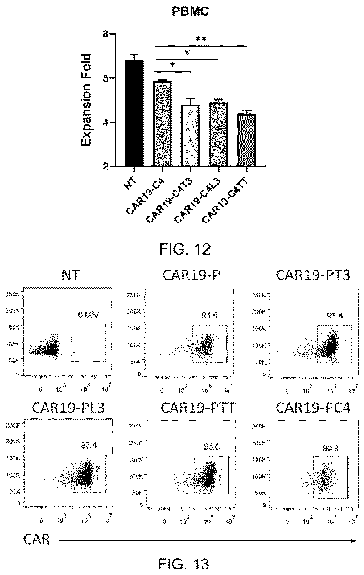

According to the detection method in 1.1.4, the suppressive effects of CAR19-C4 T, CAR19-C4T3 T, CAR19-C4L3 T and CAR19-C4TT T cells on HvGD in vitro were assessed by one-way mixed lymphocyte reaction (MLR) assay, and the results are shown in Figure 12.

It can be seen that compared with NT cells, CAR T cells expressing the immunosuppressive molecule targeting CTLA4 (CAR19-C4 T) significantly reduce the expansion fold of allogeneic PBMCs, while CAR T cells expressing the immunosuppressive molecule targeting CTLA4 and TIM3, CTLA4 and LAG3, or CTLA4 and TIGIT (CAR19-C4T3 T, CAR19-C4L3 T, or CAR19-C4TT T cells) further significantly reduce the expansion fold of allogeneic PBMCs on the basis of CAR19-C4 T cells.

The above results indicate that compared with the immunosuppressive molecule targeting only CTLA4, the immunosuppressive molecule targeting CTLA4 and other immunosuppressive proteins (such as TIM3, LAG3 or TIGIT) can significantly suppress the in vitro proliferation of allogeneic PBMCs, thereby significantly reducing HvGD, and has no obvious adverse effect on the release of cytokines (such as IL2 and IFN-γ) from CAR T cells after co-culture with target cells.

### Example 3: Construction of anti-CD19 CAR-T cells expressing the immunosuppressive molecule that comprises a PD1 binding domain and verification of the function thereof

### 3.1 Construction of anti-CD19 CAR-T cells expressing the immunosuppressive molecule comprising a PD1 binding domain

The immunosuppressive molecule linked by T2A (SEQ ID NO: 110) was further expressed in the CAR19 plasmid prepared in Example 1, and its structure was as follows: B2M signal peptide (SEQ ID NO: 94), immunosuppressive protein binding domain (Table 3), IgG4 hinge region (SEQ ID NO: 88), CD28 transmembrane region (SEQ ID NO: 82), CD28 co-stimulatory domain (SEQ ID NO: 89) to obtain plasmids CAR19-P, CAR19-PT3, CAR19-PL3, and CAR19-PC4, and the correct insertion of the target sequence in the plasmid was confirmed by sequencing.

**Table 3 CAR19 plasmids expressing the immunosuppressive molecule comprising a PD1 binding domain**

| CAR target | Immunosuppressive protein binding domain | Plasmid |
|---|---|---|
| CD19 | Anti-PD1 scFv (SEQ ID NO:77) | CAR19-P |
| CD19 | Anti-PD1 scFv (SEQ ID NO:77), linker (SEQ ID NO:111), anti-TIM3 scFv (SEQ ID NO:21) | CAR19-PT3 |
| CD19 | Anti-PD1 scFv (SEQ ID NO:77), linker (SEQ ID NO:111), anti-LAG3 scFv (SEQ ID NO:34) | CAR19-PL3 |
| CD19 | Anti-PD1 scFv (SEQ ID NO:77), linker (SEQ ID NO:111), anti-CTLA4 scFv (SEQ ID NO:64) | CAR19-PC4 |

According to the method in 1.1.1, plasmids CAR19-P, CAR19-PT3, CAR19-PL3, and CAR19-PC4 were transferred into T cells, respectively, to obtain CAR19-P T, CAR19-PT3 T, CAR19-PL3 T and CAR19-PC4 T cells.

### 3.2 Detection of CAR expression levels and immunosuppressive protein binding domain expression levels in various CAR-T cells

Using a flow cytometry, the expression of anti-CD19 scFv in CAR19-P T, CAR19-PT3 T, CAR19-PL3 T and CAR19-PC4 T cells was detected with FITC goat anti-human Fab (Thermo Fisher). It can be seen that all the CARs in the CAR-T cells prepared in this example can be effectively expressed (Figure 13).

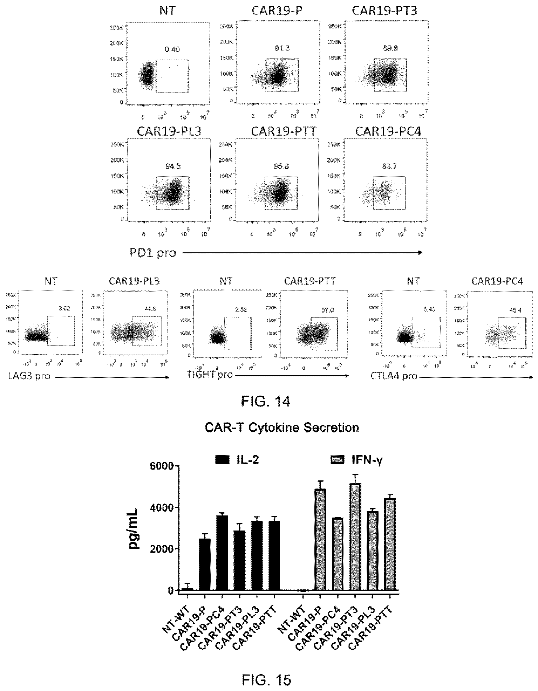

Using a flow cytometry, the expression of anti-PD1 scFv in CAR19-P T, CAR19-PT3 T, CAR19-PL3 T, and CAR19-PC4 T cells was detected with Human PD-1 / PDCD1 Protein, His Tag (ACRO); the expression of anti-LAG3 scFv in CAR19-PL3 T cells was detected with Human LAG-3 / CD223 Protein, His Tag (ACRO); the expression of anti-TIGIT scFv in CAR19-PTT T cells was detected with Human TIGIT Protein, Fc Tag (ACRO); and the expression of anti-CTLA4 scFv in CAR19-PC4 T cells was detected with Human CTLA-4 / CD152 Protein, Fc Tag (ACRO). It can be seen that anti-PD1 scFv in CAR19-P T, CAR19-PT3 T, CAR19-PL3 T, and CAR19-PC4 T cells, anti-LAG3 scFv in CAR19-PL3 T cells, anti-TIGIT scFv in CAR19-PTT T cells, and anti-CTLA4 scFv in CAR19-PC4 T cells can all be effectively expressed (Figure 14).

### 3.3 Detection of cytokine release levels in various CAR-T cells

According to the method in 1.1.3, the contents of IL2 and IFN-γ in the co-culture supernatant of CAR-T cells and target cells were detected, and the results are shown in Figure 15.

It can be seen that compared with NT cells, CAR T cells expressing the immunosuppressive molecules of the present invention (CAR19-PT, CAR19-PT3, CAR19-PL3 and CAR19-PC4 T cells) can all significantly increase the release levels of cytokines IL2 and IFN-γ, indicating that this cytokine release is specific. In addition, the cytokine release levels among CAR19-P T, CAR19-PT3 T, CAR19-PL3 T and CAR19-PC4 T cells are not much different, indicating that compared with the immunosuppressive molecule targeting only PD1, the immunosuppressive molecule targeting a combination of PD1 and other immunosuppressive proteins (TIM3, LAG3 or CTLA4) has no obvious adverse effect on the release of cytokines from CAR T cells.

### 3.4 Detection of the suppressive effects of various CAR-T cells on HvGD in vitro

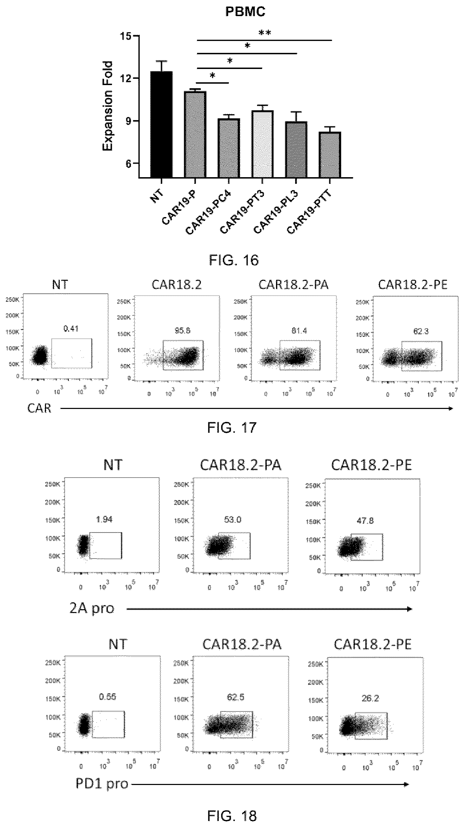

According to the detection method in 1.1.4, the suppressive effects of CAR19-P T, CAR19-PT3 T, CAR19-PL3 T and CAR19-PC4 T cells on HvGD in vitro were assessed by one-way mixed lymphocyte reaction (MLR) assay, and the results are shown in Figure 16.

It can be seen that compared with NT cells, CAR T cells expressing the immunosuppressive molecules of the present invention (CAR19-P T, CAR19-PT3 T, CAR19-PL3 T or CAR19-PC4 T cells) can all significantly suppress the proliferation of allogeneic PBMCs. More importantly, compared with CAR19-P T cells, CAR T cells expressing the immunosuppressive molecule targeting PD1 and TIM3, PD1 and LAG3, or PD1 and CTLA4 further significantly suppress the in vitro proliferation of allogeneic PBMCs.

The above results indicate that compared with the immunosuppressive molecule targeting only PD1, the immunosuppressive molecule targeting PD1 and other immunosuppressive proteins (such as TIM3, LAG3 or CTLA4) can significantly suppress the in vitro proliferation of allogeneic PBMCs, thereby significantly reducing HvGD, and has no obvious adverse effect on the release of cytokines (such as IL2 and IFN-γ) from CAR T cells after co-culture with target cells.

### Example 4: Construction of anti-Claudin 18.2 CAR-T cells expressing the immunosuppressive molecule comprising a PD1 binding domain and verification of the function thereof

### 4.1 Construction of anti-Claudin 18.2 CAR-T cells expressing the immunosuppressive molecule comprising a PD1 binding domain

Sequences encoding the following were synthesized and sequentially cloned into the pGEM-T Easy vector (Promega): anti-Claudin 18.2 VHH (SEQ ID NO: 112), CD8α hinge region (SEQ ID NO: 86), CD8α transmembrane region (SEQ ID NO: 83), 4-1BB co-stimulatory domain (SEQ ID NO: 93), CD3ζ intracellular region (SEQ ID NO: 92) to obtain a CAR18.2 plasmid, and the correct insertion of the target sequence in the plasmid was confirmed by sequencing.

The immunosuppressive molecule linked by T2A (SEQ ID NO: 110) was further expressed in the above CAR18.2 plasmid, and its structure was as follows: B2M signal peptide (SEQ ID NO: 94), immunosuppressive protein binding domain (Table 4), IgG4 hinge region (SEQ ID NO: 88), CD28 transmembrane region (SEQ ID NO: 82), CD28 co-stimulatory domain (SEQ ID NO: 89) to obtain plasmids CAR18.2-P, CAR18.2-PA and CAR18.2-PE, and the correct insertion of the target sequence in the plasmid was confirmed by sequencing.

**Table 4 CAR18.2 plasmids expressing the immunosuppressive molecule comprising a PD1 binding domain**

| CAR target | Immunosuppressive protein binding domain | Plasmid |
|---|---|---|
| Claudin 18.2 | Anti-PD1 scFv (SEQ ID NO:77) | CAR18.2-P |
| Claudin 18.2 | Anti-PD1 scFv (SEQ ID NO:77), linker (SEQ ID NO:111), anti-NKG2A scFv (SEQ ID NO:9) | CAR18.2-PA |
| Claudin 18.2 | Anti-PD1 scFv (SEQ ID NO:77), linker (SEQ ID NO:111), HLA-E extracellular region (SEQ ID NO:10) | CAR18.2-PE |

According to the method in 1.1.1, plasmids Claudin 18.2, CAR18.2-P, CAR18.2-PA and CAR18.2-PE were transferred into T cells respectively, to obtain CAR18.2 T, CAR18.2-PT, CAR18.2-PA T and CAR18.2-PE T cells.

### 4.2 Detection of CAR expression levels and immunosuppressive protein binding domain expression levels in various CAR-T cells

Using a flow cytometry, the expression of anti-Claudin 18.2 VHH in CAR18.2-P T, CAR18.2-PA T, and CAR18.2-PE T cells was detected with anti-VHH antibody (Genscript). It can be seen that the CARs in CAR18.2-P T, CAR18.2-PA T, and CAR18.2-PE T cells can all be effectively expressed (Figure 17).

Using a flow cytometry, the expression of anti-NKG2A scFv or HLA-E in CAR18.2-PA T and CAR18.2-PE T cells was detected with Human NKG2A&CD94 Protein, Fc Tag (ACRO); and the expression of anti-PD1 scFv in CAR18.2-PA T and CAR18.2-PE T cells was detected with Human PD-1 / PDCD1 Protein, His Tag (ACRO). It can be seen that anti-NKG2A scFv, HLA-E, and anti-PD1 scFv in CAR18.2-PA T and CAR18.2-PE T cells can all be effectively expressed (Figure 18).

### 4.3 Detection of the killing effects of various CAR-T cells on target cells

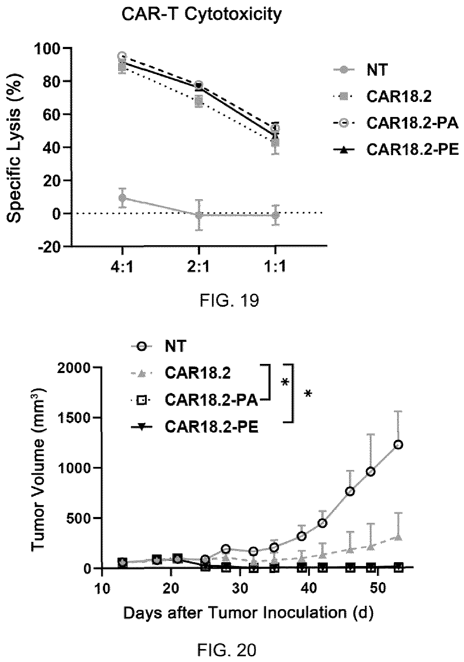

Target cells Claudin 18.2/NUGC4 were plated into 96-well plates at a concentration of 1×10⁴ cells/well, and then, NT, CAR18.2 T, CAR18.2-PA T, and CAR18.2-PE T cells were plated into the 96-well plates at an effector-to-target ratio of 4:1, 2:1 or 1:1 for co-culture. After 18 hours, the fluorescence value was measured with a microplate reader. According to the calculation formula: (average fluorescence value of target cells - average fluorescence value of samples)/average fluorescence value of target cells × 100%, the killing efficiency was calculated, and the results are shown in Figure 19.

It can be seen that CAR18.2 T, CAR18.2-PA T and CAR18.2-PE T cells at different effector-to-target ratios all show effective specific killing effects on target cells, and the killing effects are comparable, indicating that the additional expression of the immunosuppressive molecule targeting PD1 and NKG2A has no obvious effect on the killing activity of CAR18.2 T.

### 4.4 Detection of in vivo suppressive effect of various CAR T cells on tumors

Twenty-five healthy female NPI mice aged 6-8 weeks were divided into 4 groups, with 5 mice in each group: NT group, CAR18.2 T group, CAR18.2-PA T group, and CAR18.2-PE T group. On day 0, 5×10⁵ Claudin 18.2/NUGC4 cells were injected into the tail vein of each mouse. Six days later, 2× 10 ⁶ NT cells or corresponding CAR-T cells were injected into the tail vein of each mouse according to the grouping. The status of the mice was assessed weekly. Changes in tumor volume in the mice is shown in Figure 20.

It can be seen that the tumor suppressive effects of the CAR18.2-PA T group and the CAR18.2-PE T group are significantly higher than those of the CAR18.2 T and NT groups, indicating that the immunosuppressive molecule targeting PD1 and NKG2A can very effectively suppress immune rejection in vivo, thereby enhancing the tumor suppressive effect of CAR-T cells. In addition, the tumor suppressive effects of the CAR18.2-PA T group and the CAR18.2-PE T group are comparable, suggesting that different structural forms (such as antibodies or ligands) used in the immunosuppressive protein (such as NKG2A) binding domain in the immunosuppressive molecule of the present invention are capable of significantly suppressing immune rejection and exerting anti-tumor effects.

### Example 5: Construction of anti-Claudin 18.2 CAR-T cells expressing the immunosuppressive molecule and verification of the function thereof

### 5.1 Construction of anti-Claudin 18.2 CAR-T cells expressing the immunosuppressive molecule

Sequences encoding the following were synthesized and sequentially cloned into the pGEM-T Easy vector (Promega): anti-Claudin 18.2 VHH (SEQ ID NO: 112), CD8α hinge region (SEQ ID NO: 86), CD8α transmembrane region (SEQ ID NO: 83), 4-1BB co-stimulatory domain (SEQ ID NO: 93), CD3ζ intracellular region (SEQ ID NO: 92) to obtain a CAR18.2 plasmid, and the correct insertion of the target sequence in the plasmid was confirmed by sequencing.

The immunosuppressive molecule linked by T2A (SEQ ID NO: 110) was further expressed in the above CAR18.2 plasmid, and its structure was as follows: B2M signal peptide (SEQ ID NO: 94), immunosuppressive protein binding domain (Table 5), IgG4 hinge region (SEQ ID NO: 88), CD28 transmembrane region (SEQ ID NO: 82), CD28 co-stimulatory domain (SEQ ID NO: 89) to obtain plasmids CAR18.2-PC, CAR18.2-CA, CAR18.2PTT, CAR18.2-PL3, and CAR18.2-PT3, and the correct insertion of the target sequence in the plasmid was confirmed by sequencing.

**Table 5 CAR18.2 plasmids expressing the immunosuppressive molecule**

| CAR target | Immunosuppressive protein binding domain | Plasmid |
|---|---|---|
| Claudin 18.2 | PDL1 extracellular region (SEQ ID NO:78), linker (SEQ ID NO:111), anti-CTLA4 scFv (SEQ ID NO:64) | CAR18.2-PC |
| Claudin 18.2 | Anti-CTLA4 scFv (SEQ ID NO:64), linker (SEQ ID NO:111), anti-NKG2A scFv (SEQ ID NO:9) | CAR18.2-CA |
| Claudin 18.2 | PDL1 extracellular region (SEQ ID NO:78), linker (SEQ ID NO:111), anti-TIGIT scFv (SEQ ID NO:47) | CAR18.2PTT |
| Claudin 18.2 | PDL1 extracellular region (SEQ ID NO:78), linker (SEQ ID NO:111), anti-LAG3 scFv (SEQ ID NO:34) | CAR18.2-PL3 |
| Claudin 18.2 | PDL1 extracellular region (SEQ ID NO:78), linker (SEQ ID NO:111), anti-TIM3 scFv (SEQ ID NO:21) | CAR18.2-PT3 |

According to the method in 1.1.1, plasmids Claudin 18.2, CAR18.2-PC, CAR18.2-CA, CAR18.2PTT, CAR18.2-PL3 and CAR18.2-PT3 were transferred into T cells respectively, to obtain CAR18.2 T, CAR18.2-PC T, CAR18.2-CA T, CAR18.2PTT T, CAR18.2-PL3 T, CAR18.2-PT3 T cells.

### 5.2 Detection of CAR expression levels and immunosuppressive protein binding domain expression levels in various CAR-T cells

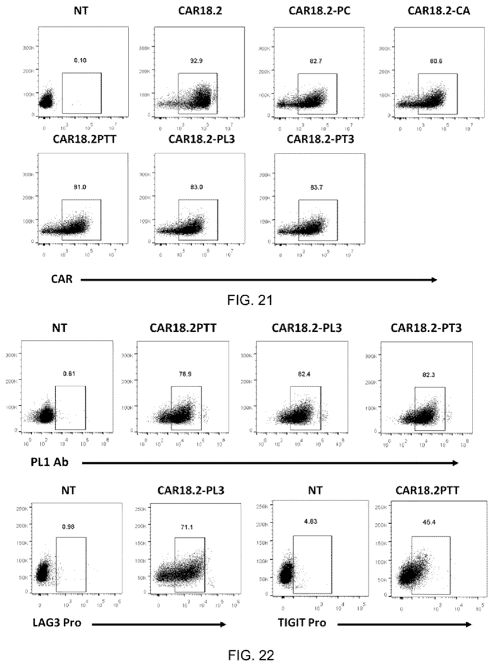

Using a flow cytometry, the expression of anti-Claudin 18.2 VHH in CAR18.2 T, CAR18.2-PC T, CAR18.2-CA T, CAR18.2PTT T, CAR18.2-PL3 T, and CAR18.2-PT3 T cells was detected with anti-VHH antibody (Genscript). It can be seen that all the CARs in various CAR-T cells can be effectively expressed (Figure 21).

Using a flow cytometry, the expression of PDL1 in CAR18.2-PC T, CAR18.2-CAT, CAR18.2PTT T, CAR18.2-PL3 T, and CAR18.2-PT3 T cells was detected with Human PD-1 / PDCD1 Protein, His Tag (ACRO), and it can be seen that PDL1 in CAR18.2-PC T, CAR18.2-CAT, CAR18.2PTT T, CAR18.2-PL3 T, and CAR18.2-PT3 T cells can be normally expressed (Figure 22). The expression of anti-LAG3 scFv in CAR18.2-PL3 T cells was detected with Human LAG-3 / CD223 Protein, His Tag (ACRO), and the expression of anti-TIGIT scFv in CAR18.2-PTT T cells was detected with Human TIGIT Protein, Fc Tag (ACRO). It can be seen that both anti-LAG3 scFv in CAR18.2-PL3 T cells and anti-TIGIT scFv in CAR18.2-PTT T cells can be effectively expressed (Figure 22).

### 5.3 Detection of the killing effects of various CAR-T cells on target cells

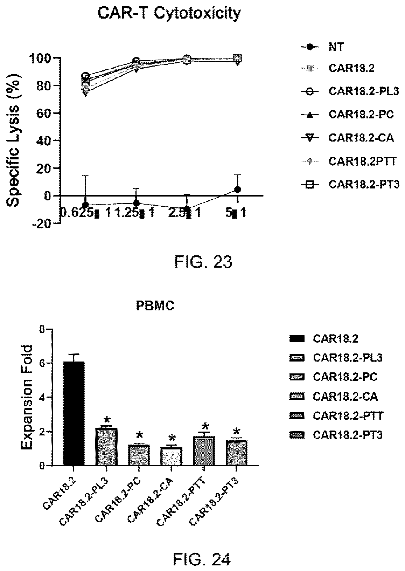

Target cells Claudin 18.2/NUGC4 were plated into 96-well plates at a concentration of 1×10⁴ cells/well, and then, NT, CAR18.2-PC T, CAR18.2-CA T, CAR18.2PTT T, CAR18.2-PL3 T, and CAR18.2-PT3 T cells were plated into the 96-well plates at an effector-to-target ratio of 0.625:1, 1.25:1, 2.5:1 or 5:1 for co-culture. After 18 hours, the fluorescence value was measured with a microplate reader. According to the calculation formula: (average fluorescence value of target cells - average fluorescence value of samples)/average fluorescence value of target cells × 100%, the killing efficiency was calculated, and the results are shown in Figure 23.

It can be seen that all the T cells at different effector-to-target ratios show effective specific killing effects on target cells, and the killing effects are comparable, indicating that the additional expression of the immunosuppressive molecule of the present invention has no obvious effect on the killing activity of CAR18.2 T.

### 5.4 Detection of the suppressive effects of CAR-T on HvGD in vitro

The suppressive effects of various CAR-T cells prepared in 5.1 on HvGD in vitro were assessed by one-way mixed lymphocyte reaction (MLR) assay. Specifically, CAR18.2 T, CAR18.2-PC T, CAR18.2-CA T, CAR18.2PTT T, CAR18.2-PL3 T, CAR18.2-PT3 T cells were co-incubated with allogeneic PBMCs under 37°C and 5% CO₂ for 10 days, and the proliferation of PBMCs was then detected by flow cytometry, and the results are shown in Figure 24.

It can be seen that compared with CAR18.2 T cells, CAR18.2-PC T, CAR18.2-CA T, CAR18.2PTT T, CAR18.2-PL3 T, and CAR18.2-PT3 T can significantly suppress the proliferation of allogeneic PBMCs
In summary, among the tested immunosuppressive proteins NKG2A, TIM3, LAG3, TIGIT, CTLA4, and PD1, compared with the expression of the immunosuppressive molecule targeting only one immunosuppressive protein or expressing no immunosuppressive molecules, the expression of the immunosuppressive molecule targeting a combination of multiple (e.g., two) immunosuppressive proteins (e.g., targeting NKG2A and TIM3, NKG2A and LAG3, NKG2A and TIGIT, NKG2A and CTLA4, CTLA4 and TIM3, CTLA4 and LAG3, CTLA4 and TIGIT, PD1 and TIM3, PD1 and LAG3, PD1 and TIGIT, PD1 and CTLA4, or PD1 and NKG2A), on the one hand, will have no obvious adverse effect on the killing activity or the cytokine release characteristic of CAR-T cells, and on the other hand, it can greatly enhance the suppressive effect of the killing activity of allogeneic NK cells or the excessive proliferation of allogeneic PBMCs after being activated, thereby effectively reducing the rejection responses of immune cells.

It should be noted that the above-mentioned are merely for preferred examples of the present invention and not used to limit the present invention. For those skilled in the art, various modifications and changes may be made to the present invention. Those skilled in the art should understand that any amendments, equivalent replacements, improvements, and so on, made within the spirit and principle of the present invention, should be covered within the scope of protection of the present invention.

## Claims

1. An immunosuppressive molecule comprising an immunosuppressive protein binding domain, a transmembrane domain and a co-stimulatory domain and the molecule does not comprise a primary signaling domain, **characterized in that** the immunosuppressive protein is selected from two or more of the group consisting of: NKG2A, TIM3, LAG3, TIGIT, CTLA4, PD1 and FasL.

2. The immunosuppressive molecule according to claim 1, **characterized in that** the immunosuppressive protein binding domain is selected from the group consisting of an intact antibody, Fab, Fab', F(ab')2, an Fd fragment, Fd', an Fv fragment, scFv, sdFv, a linear antibody, a diabody, sdAbs, and a functional fragment of a ligand or a receptor.

3. The immunosuppressive molecule according to any one of claims 1-2, **characterized in that** the immunosuppressive molecule binds to any one of the following combinations of immunosuppressive proteins: NKG2A and TIM3, NKG2A and LAG3, NKG2A and TIGIT, NKG2A and CTLA4, CTLA4 and TIM3, CTLA4 and LAG3, CTLA4 and TIGIT, PD1 and TIM3, PD1 and LAG3, PD1 and TIGIT, PD1 and CTLA4, TIM3 and LAG3, TIM3 and TIGIT, LAG3 and TIGIT, PD1 and NKG2A, FasL and NKG2A, FasL and LAG3, FasL and TIGIT, FasL and CTLA4, or FasL and PD1.

4. The immunosuppressive molecule according to any one of claims 1-3, **characterized in that** the immunosuppressive molecule comprises: (1) an immunosuppressive protein binding region comprising a first immunosuppressive protein binding domain and a second immunosuppressive protein binding domain; (2) a transmembrane domain; and (3) a co-stimulatory domain.

5. The immunosuppressive molecule according to any one of claims 1-3, **characterized in that** the immunosuppressive molecule comprises: (1) a first unit structure targeting a first immunosuppressive protein, comprising a first immunosuppressive protein binding domain, a transmembrane domain, and a co-stimulatory domain; and (2) a second unit structure targeting a second immunosuppressive protein, comprising a second immunosuppressive protein binding domain, a transmembrane domain, and a co-stimulatory domain.

6. The immunosuppressive molecule according to any one of claims 1-5, **characterized in that** the immunosuppressive molecule comprises two or more of an NKG2A binding domain, a TIM3 binding domain, an LAG3 binding domain, a TIGIT binding domain, a PD1 binding domain, a CTLA4 binding domain, and an FasL binding domain; wherein,
(1) the NKG2A binding domain is an NKG2A-targeting antibody, a ligand, or a functional fragment thereof;
(2) the TIM3 binding domain is a TIM3-targeting antibody, a ligand, or functional fragment thereof;
(3) the LAG3 binding domain is an LAG3-targeting antibody,a ligand, or a functional fragment thereof;
(4) the TIGIT binding domain is a TIGIT-targeting antibody,a ligand, or a functional fragment thereof;
(5) the CTLA4 binding domain is a CTLA4-targeting antibody, a ligand, or functional fragment thereof; or
(6) the PD1 binding domain is a PD1-targeting antibody, a ligand, or a functional fragment thereof;
(7) the FasL binding domain is an FasL-targeting antibody, a ligand, or a functional fragment thereof.

7. The immunosuppressive molecule according to claim 6, **characterized in that**:
(1) the NKG2A-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 7; and the CDR1-L, CDR2-L and CDR3-L comprised in the light chain variable region are the same as the CDR1-L, CDR2-L and CDR3-L comprised in SEQ ID NO: 8; preferably, the heavy chain variable region comprises an amino acid sequence having at least 90% identity to SEQ ID NO:7, and the light chain variable region comprises an amino acid sequence having at least 90% identity to SEQ ID NO:8;
(2) the TIM3-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 19; and the CDR1-L, CDR2-L and CDR3-L comprised in the light chain variable region are the same as the CDR1-L, CDR2-L and CDR3-L comprised in SEQ ID NO: 20; preferably, the heavy chain variable region comprises an amino acid sequence having at least 90% identity to SEQ ID NO: 19, and the light chain variable region comprises an amino acid sequence having at least 90% identity to SEQ ID NO: 20;
(3) the LAG3-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 32; and the CDR1-L, CDR2-L and CDR3-L comprised in the light chain variable region are the same as the CDR1-L, CDR2-L and CDR3-L comprised in SEQ ID NO: 33; preferably, the heavy chain variable region comprises an amino acid sequence having at least 90% identity to SEQ ID NO: 32, and the light chain variable region comprises an amino acid sequence having at least 90% identity to SEQ ID NO: 33;
(4) the TIGIT-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 45; and the CDR1-L, CDR2-L and CDR3-L comprised in the light chain variable region are the same as the CDR1-L, CDR2-L and CDR3-L comprised in SEQ ID NO: 46; preferably, the heavy chain variable region comprises an amino acid sequence having at least 90% identity to SEQ ID NO:45, and the light chain variable region comprises an amino acid sequence having at least 90% identity to SEQ ID NO:46;
(5) the CTLA4-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 62; and the CDR1-L, CDR2-L and CDR3-L comprised in the light chain variable region are the same as the CDR1-L, CDR2-L and CDR3-L comprised in SEQ ID NO: 63; preferably, the heavy chain variable region comprises an amino acid sequence having at least 90% identity to SEQ ID NO:62, and the light chain variable region comprises an amino acid sequence having at least 90% identity to SEQ ID NO:63; or
(6) the PD1-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 75; and the CDR1-L, CDR2-L and CDR3-L comprised in the light chain variable region are the same as the CDR1-L, CDR2-L and CDR3-L comprised in SEQ ID NO: 76; preferably, the heavy chain variable region comprises an amino acid sequence having at least 90% identity to SEQ ID NO: 75, and the light chain variable region comprises an amino acid sequence having at least 90% identity to SEQ ID NO: 76;
(7) the FasL-targeting antibody comprises a light chain variable region and a heavy chain variable region, wherein the CDR1-H, CDR2-H and CDR3-H comprised in the heavy chain variable region are the same as the CDR1-H, CDR2-H and CDR3-H comprised in SEQ ID NO: 119 or 125; wherein the CDR1-L, CDR2-L and CDR3-L comprised in the light chain variable region are the same as the CDR1-L, CDR2-L and CDR3-L comprised in SEQ ID NO: 120; preferably, the heavy chain variable region comprises an amino acid sequence having at least 90% identity to SEQ ID NO: 119 or 125, and the light chain variable region comprises an amino acid sequence having at least 90% identity to SEQ ID NO: 120.

8. The immunosuppressive molecule according to claim 6, **characterized in that**:
(1) the NKG2A-targeting ligand comprises an amino acid sequence having at least 90% identity to SEQ ID NO: 10 or 11;
(2) the TIM3-targeting ligand comprises an amino acid sequence having at least 90% identity to SEQ ID NO: 22, 23 or 24;
(3) the LAG3-targeting ligand comprises an amino acid sequence having at least 90% identity to SEQ ID NO: 35, 36 or 37;
(4) the TIGIT-targeting ligand comprises an amino acid sequence having at least 90% identity to SEQ ID NO: 48, 49, 50 or 51;
(5) the CTLA4-targeting ligand comprises an amino acid sequence having at least 90% identity to SEQ ID NO: 65 or 66;
(6) the PD1-targeting ligand comprises an amino acid sequence having at least 90% identity to SEQ ID NO: 78 or 79;
(7) the FasL-targeting receptor comprises an amino acid sequence having at least 90% identity to SEQ ID NO: 127.

9. The immunosuppressive molecule according to any one of claims 1-8, **characterized in that** the transmembrane domain is selected from the transmembrane domains of the proteins consisting of: a TCR α chain, a TCR β chain, a TCR γ chain, a TCR δ chain, a CD3 ζ subunit, a CD3 ε subunit, a CD3 γ subunit, a CD3 δ subunit, CD28, CD45, CD4, CD5, CD8α, CD9, CD16, CD22, CD33, CD37, CD47, CD64, CD80, CD86, CD94, CD112, CD113, CD134, CD137, CD154, CD155, Nectin4, KIRDS2, OX40, CD2, CD27, CD18, ICOS, 4-1BB, GITR, CD40, BAFFR, HVEM, SLAMF7, NKp80, CD160, BCMA, IL-2R β, IL-2R γ, IL-7R a, ITGA1, VLA1, CD49a, ITGA4, IA4, CD49D, ITGA6, VLA-6, CD49f, ITGAD, CD1 ld, ITGAE, CD103, ITGAL, CDl 1a, ITGAM, CDl lb, ITGAX, CDl lc, ITGB1, CD29, ITGB2, CD18, LFA-1, ITGB7, TNFR2, DNAM1, SLAMF4, CD84, CD96, HLA-E, LSECtin, PDL1, PDL2, CEACAM1, Fas, MHC class II molecules, CRT AM, Ly9, CD160, PSGL1, CDIOO, SLAMF6, SLAMF1, SLAMF8, CD162, LTBR, PAG/Cbp, NKp44, NKp30, NKp46, NKG2D and NKG2C.

10. The immunosuppressive molecule according to any one of claims 1-9, **characterized in that** the co-stimulatory domain is selected from the intracellular regions of the proteins consisting of: LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, 4-1BB, CD270, CD272, B7-H3, ICOS, CD357, DAP10, DAP12, LAT, NKG2C, SLP76, PD1, LIGHT, TRIM, ZAP70, and any combination thereof.

11. The immunosuppressive molecule according to any one of claims 1-10, **characterized in that** the primary signaling domain is selected from the intracellular regions of the proteins consisting of: FcRγ, FcRβ, CD3γ, CD3δ, CD3ε, CD3ζ, CD5, CD22, CD79a, CD79b, NFAM1, STAM1, STAM2, and Cd66d.

12. The immunosuppressive molecule according to any one of claims 1-11, further comprising a signal peptide.

13. The immunosuppressive molecule according to claims 1-12, further comprising a hinge region between the immunosuppressive protein binding domain and the transmembrane domain, the hinge region derived from the group consisting of: CD8α, CD28, FcγRIIIα receptor, IgG4 or IgG1.

14. A nucleic acid molecule encoding the immunosuppressive molecule according to any one of claims 1-13.

15. A vector comprising the nucleic acid according to claim 14.

16. An engineered cell expressing the immunosuppressive molecule according to any one of claims 1-13, or the nucleic acid molecule according to claim 14, or the vector according to claim 15.

17. The engineered cell according to claim 16, **characterized in that** the engineered cell further expresses a functional exogenous receptor that specifically recognizes an antigen.

18. The engineered cell according to claim 17, **characterized in that** the functional exogenous receptor is selected from the group consisting of: a chimeric antigen receptor, a T cell receptor, a T cell receptor fusion protein, a T cell antigen coupler, and an immune mobilizing monoclonal T cell receptor, preferably a chimeric antigen receptor or a T cell receptor.

19. The engineered cell according to claim 18, **characterized in that** the antigen specifically recognized by the functional exogenous receptor is selected from the group consisting of: ALK, ADRB3, AKAP-4, APRIL, ASGPR1, BCMA, B7H3, B7H4, B7H6, bcr-abl, BORIS, BST2, BAFF-R, BTLA, CD2, CD3, CD4, CD5, CD7, CD8, CD19, CD20, CD22, CD24, CD25, CD28, CD30, CD33, CD38, CD40, CD44, CD44v6, CD44v7/8, CD47, CD52, CD56, CD57, CD58, CD70, CD72, CD79a, CD79b, CD80, CD81, CD86, CD97, CD123, CD133, CD137, CD 138, CD151, CD171, CD179a, CD300LF, CDH16, CSPG4, CS1, Claudin 6, Claudin18.1, Claudin 18.2, CEA, CEACAM6, CLL1, c-Met, CAIX, CXORF61, CA125, CYP1B1, CS1, ELF2M, EGFR, EPCAM, EGFRvIII, EphA2, ERG/TMPRSS2ETS fusion gene, ETV6-AML, EMR2, EGP2, EGP40, FAP, FAR, FBP, FLT3, FOSL1, FCRL5, FCAR, Flt3, Flt4, Frizzled, GD2, GD3, gp100, gp130, GM3, GPC2, GPC3, GPRC5D, GPR20, GloboH, GHRHR, GHR, GITR, Her2, HER3, HER-4, HMWMAA, HAVCR1, HPV E6,E7, HVEM, HIV-1Gag, HLA-A1, HLA-A2, IL6R, IL-11Ra, IL-13Ra, IGF-I receptor, LTPR, LIFRP, LRP5, IGLL1, IGF1R, KIT, Kappa Light Chain, KDR, LewisY, LMP2, LY6K, LAGE-1a, legumain, LCK, LAIR1, LILRA2, LY75, MSLN, MUC1, MUC16, MAGE-A1, MAGE3, MAD-CT-1, MelanA/MART1, ML-IAP, MYCN, mut hsp70-2, NCAM, NY-BR-1, NY-ESO-1, NA17, Notch-1-4, nAchR, NKG2D, NKG2D ligand, OY-TES1, OR51E2, OX40, PRSS21, PSCA, PD1, PD-L1, PD-L2, PSMA, Prostase, PAP, PDGFR-β, PCTA-1/galectin 8, p53, p53 mutant, prostein, PLAC1, PANX3, PAX3, PAX5, PTCH1, RANK, RAGE-1, ROR1, Ras mutant, RhoC, RU1, RU2, Robol, SSEA-4, SSX2, SART3, Sp17, TSHR, Tn Ag, TGS5, TEM1/CD248, TEM7R, TARP, TCRα, TCRβ, TGFBR1, TGFBR2, TNFRSF4, TWEAK-R, TLR7, TLR9, TAG72, TROP-2, Tie 2, TRP-2, TNFR1, TNFR2, TEM1, UPK2VEGFR, WT1, XAGE1, 5T4, 8H9, αvβ6 integrin, CA9, folate receptor α, ephrin B2, tyrosinase, fucosyl GM1, o-acetyl-GD2, folate receptor β, polysialic acid, sperm protein 17, survivin and telomerase, sarcoma translocation breakpoint, human telomerase reverse transcriptase/hTERT, androgen receptor, intestinal carboxylesterase, cyclin B1, fibronectin, tenascin, carcinoembryonic variant of tumor necrosis region, and any combination thereof.

20. The engineered cell according to any one of claims 16-19, **characterized in that** in the engineered cell, expression of one or more genes among endogenous HLA class I gene, HLA class II gene, and TCR/CD3 gene is suppressed or silenced.

21. The engineered cell according to claim 20, **characterized in that** the HLA class I gene is selected from the group consisting of HLA-A, HLA-B, HLA-C, B2M and any combination thereof; the HLA class II gene is selected from the group consisting of HLA-DPA, HLA-DQ, HLA-DRA, TAP1, TAP2, LMP2, LMP7, RFX5, RFXAP, RFXANK, CIITA and any combination thereof; and the TCR/CD3 gene is selected from the group consisting of TRAC, TRBC, CD3γ, CD3δ, CD3ε, CD3ζ and any combination thereof.

22. The engineered cell according to any one of claims 16-21, **characterized in that** expression of one or more endogenous genes selected from the group consisting of: CD52, GR, dCK, PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3 is suppressed or silenced.

23. The engineered cell according to any one of claims 16-22, **characterized in that** the cell is an immune cell, and the cell selected from the group consisting of a B cell, a T cell, a macrophage, a dendritic cell, a monocyte, an NK cell, and an NKT cell.

24. The engineered cell according to claim 23, **characterized in that** the cell is selected from the group consisting of a CD4+CD8+ T cell, a CD4+ T cell, a CD8+ T cell, a CD4-CD8- T cell, a tumor infiltrating cell, a memory T cell, a naive T cell, a γδ-T cell and an αβ-T cell.

25. The engineered cell according to any one of claim 16-22, **characterized in that** the cell is a stem cell, the stem cell being selected from the group consisting of an embryonic stem cell, an umbilical cord blood stem cell, a bone marrow stem cell, a hematopoietic stem cell, a mesenchymal stem cell and an induced pluripotent stem cell.

26. A method for reducing immune rejection of exogenous cells by cells of a subject, **characterized by** expressing the immunosuppressive molecule of any one of claims 1-13, the nucleic acid molecule of claim 14 or the vector of claim 15 in the exogenous cells.

27. A method for reducing immune rejection of exogenous cells by cells of a subject, **characterized by** administering the immunosuppressive molecule of any one of claims 1-13 or the engineered cell of any one of claims 16-25 to the subject.

28. A pharmaceutical composition comprising the immunosuppressive molecule of any one of claims 1-13, the nucleic acid molecule of claim 14, the vector of claim 15 or the engineered cell of any one of claims 16-25, and one or more pharmaceutically acceptable excipients.

29. Use of the engineered cell of any one of claims 16-25 or the pharmaceutical composition of claim 28 in the preparation of a medicament for the prevention or treatment of cancer, infection or autoimmune diseases.
